(19) Europäisches Patentamt
European Patent Office
Office européen des brevets

(11) **EP 4 563 687 A1**

(12) **EUROPEAN PATENT APPLICATION**
published in accordance with Art. 153(4) EPC

(43) Date of publication:
04.06.2025 Bulletin 2025/23

(21) Application number: 23850111.8

(22) Date of filing: 02.08.2023

(51) International Patent Classification (IPC):
*C12M 1/00* (2006.01)   *C12M 3/00* (2006.01)

(52) Cooperative Patent Classification (CPC):
B01J 20/264; B01J 20/3274; B01J 20/3278;
C12M 1/00; C12M 3/00

(86) International application number:
PCT/JP2023/028254

(87) International publication number:
WO 2024/029557 (08.02.2024 Gazette 2024/06)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 02.08.2022 JP 2022123245

(71) Applicants:
• Terumo Kabushiki Kaisha
Tokyo 151-0072 (JP)

• Terumo BCT, Inc.
Lakewood, CO 80215 (US)

(72) Inventors:
• IGARASHI, Masatsugu
Ashigarakami-gun, Kanagawa 259-0151 (JP)
• TAKEDA, Norihiko
Ashigarakami-gun, Kanagawa 259-0151 (JP)
• SETHI, Dalip
Lakewood, Colorado 80215 (US)

(74) Representative: TBK
Bavariaring 4-6
80336 München (DE)

(54) **SEPARATING DEVICE AND SEPARATING METHOD**

(57) A controller 44 fixes an antibody 30 or an aptamer to a surface 26 of a base material 20 of a filter 10 by operating a pump 78 in a state where a first valve (76c, 76d) is opened and a second valve (76a) is closed to cause a first liquid to pass through the base material 20, and captures a separation target with the antibody 30 or the aptamer by operating, after fixing the antibody 30 or the aptamer to the surface 26 of the base material 20, the pump 78 in a state where the first valve is closed and the second valve is opened to cause a second liquid to pass through the base material 20.

FIG. 3

VALVE: OPENED
VALVE: CLOSED

CONTROLLER 44

EP 4 563 687 A1

## Description

Technical Field

The present invention relates to a separation device and a separation method for separating a separation target from a liquid.

Background Art

Examples of a method for separating a cell from a cell suspension include a method using a filter and a centrifugation method. For example, JP 2011-24575 A discloses a centrifugation method.

Summary of Invention

Technical Problem

Some cell suspensions contain a plurality of types of cells having similar sizes. In a method using a filter having fine pores and a centrifugation method, it is difficult to separate a specific cell from a plurality of types of cells having similar sizes.

An object of the present invention is to solve the above-described problem.

Solution to Problem

A separation device of a first invention is (1) a separation device that separates a separation target from a liquid, the separation device including: a pump capable of causing a first liquid containing an antibody or an aptamer capable of specifically binding to an antigen of the separation target and a second liquid containing the separation target to pass through a base material made of a porous polyester or a porous polyurethane capable of binding to the antibody or the aptamer; a first valve capable of opening and closing a first flow path through which the first liquid flows; a second valve capable of opening and closing a second flow path through which the second liquid flows; and a controller that controls operation of the pump, opening and closing of the first valve, and opening and closing of the second valve, in which the controller fixes the antibody or the aptamer to a surface of the base material by operating the pump in a state where the first valve is opened and the second valve is closed to cause the first liquid to pass through the base material, and captures the separation target with the antibody or the aptamer by operating, after fixing the antibody or the aptamer to the surface of the base material, the pump in a state where the first valve is closed and the second valve is opened to cause the second liquid to pass through the base material.

According to the above configuration, a specific cell can be separated from a plurality of types of cells having similar sizes contained in a cell liquid, and similarly, a specific protein can be separated from a plurality of types of proteins having similar sizes. Furthermore, according to the above configuration, the treatment of manufacturing the base material as a filter and the treatment of separating the separation target from the cell liquid can be performed by the same device. Therefore, the work can be efficiently performed.

(2) The device of the first invention preferably includes: a first container that stores the first liquid; a second container that stores the second liquid; and a third container that accommodates the base material, in which the controller causes the first container and the third container to communicate with each other by opening the first valve and closing the second valve, and causes the second container and the third container to communicate with each other by closing the first valve and opening the second valve after fixing the antibody or the aptamer to the surface of the base material.

(3) In the device according to (1) or (2), preferably, the third container accommodates a plurality of the base materials, and a plurality of the base materials is stacked from an upstream side to a downstream side of a flow of the first liquid and the second liquid.

According to the above configuration, the cell liquid can be brought into contact with each of the base materials.

(4) The device according to any one of (1) to (3) preferably includes a plurality of the third containers arranged in a line from an upstream side to a downstream side of a flow of the first liquid and the second liquid, in which a type of the antibody or the aptamer fixed to the base material is different among the third containers.

According to the above configuration, a plurality of types of separation targets can be separated by causing the cell liquid to flow through the third container once.

(5) In the separation device according to any one of (1) to (4), preferably, biotin is fixed to a surface of the base material via a constituent unit A derived from a compound A having an epoxy group and an ethylenically unsaturated group and a constituent unit B derived from a diamine compound B binding to a terminal of the constituent unit A, the constituent unit A binds to the base material, and the constituent unit B binds to the biotin.

(6) In the separation device according to claim (5), the compound A is preferably at least one selected from the group consisting of glycidyl acrylate, glycidyl methacrylate (GMA), 3,4-epoxycyclohexyl methyl acrylate, 3,4-epoxycyclohexyl methyl methacrylate, β-methyl glycidyl acrylate, and β-methyl glycidyl methacrylate.

(7) In the separation device according to (5) or (6), the compound B is preferably at least one selected from the group consisting of ethylenediamine, trimethylenediamine, 1,2-diaminopropane, tetramethylenediamine, 1,3-diaminobutane, 2,3-diaminobutane, pentamethylenediamine, 2,4-diaminopentane, hexamethylenediamine (HMDA), octamethylenediamine, nonamethylenediamine, decamethylenediamine, undecamethylenediamine, dodecamethylenediamine, tridecamethylenediamine, octadecamethylenediamine, xylylenediamine, and phenylenediamine.

A second invention is (8) a separation method for separating a separation target from a liquid, the separation method including: a first step of fixing an antibody or an aptamer capable of specifically binding to an antigen of the separation target to a surface of a base material made of a porous polyester or a porous polyurethane capable of binding to the antibody or the aptamer by causing a first liquid containing the antibody or the aptamer to pass through the base material, and a second step of capturing the separation target with the antibody or the aptamer by causing a second liquid containing the separation target to pass through the base material after the first step.

According to the above configuration, the same effect as that of the first invention can be obtained.

(9) In the method of the second invention, preferably, a separation device including: a first container that stores the first liquid; a second container that stores the second liquid; a third container that accommodates the base material; a pump capable of causing the first liquid and the second liquid to flow to the base material; a first valve capable of opening and closing a first flow path through which the first liquid flows; a second valve capable of opening and closing a second flow path through which the second liquid flows; and a controller that controls operation of the pump, opening and closing of the first valve, and opening and closing of the second valve is used, and the controller causes the first container and the third container to communicate with each other by opening the first valve and closing the second valve in the first step, and causes the second container and the third container to communicate with each other by closing the first valve and opening the second valve in the second step.

Advantageous Effects of Invention

According to the present invention, a specific cell can be separated from a plurality of types of cells having similar sizes, and similarly, a specific protein can be separated from a plurality of types of proteins having similar sizes.

Brief Description of Drawings

Fig. 1 is a diagram illustrating a configuration of a filter.
Figs. 2A to 2C are partially enlarged diagrams of a filter unit.
Fig. 3 is a diagram illustrating a configuration of a separation device.
Fig. 4 is a flowchart of a filter manufacturing treatment and a separation treatment performed using the separation device.
Fig. 5 is a diagram illustrating opening and closing of a valve of the separation device and a flow of a liquid in the separation device in steps S1, S2, and S5 of Fig. 4.
Fig. 6 is a diagram illustrating opening and closing of the valve of the separation device and a flow of the liquid in the separation device in step S1 of Fig. 4.
Fig. 7 is a diagram illustrating opening and closing of the valve of the separation device and a flow of the liquid in the separation device in step S2 of Fig. 4.
Fig. 8 is a diagram illustrating opening and closing of the valve of the separation device and a flow of the liquid in the separation device in step S3 of Fig. 4.
Fig. 9 is a diagram illustrating opening and closing of the valve of the separation device and a flow of the liquid in the separation device in step S3 of Fig. 4.
Fig. 10 is a diagram illustrating opening and closing of the valve of the separation device and a flow of the liquid in the separation device in step S4 of Fig. 4.
Fig. 11 is a diagram illustrating opening and closing of the valve of the separation device and a flow of the liquid in the separation device in step S4 of Fig. 4.
Fig. 12 is a diagram illustrating opening and closing of the valve of the separation device and a flow of the liquid in the separation device in step S5 of Fig. 4.
Fig. 13 is a diagram illustrating opening and closing of the valve of the separation device and a flow of the liquid in the separation device in a cell collection step.
Fig. 14 is a diagram for describing fixation of an active group capable of specifically binding to a separation target according to the present invention.

Fig. 15 is a fluorescent photograph indicating presence of biotin in sample 1 in Example 1.

Fig. 16 is a schematic diagram illustrating a cell capture evaluating test device.

Description of Embodiments

One aspect of the present invention provides a separation device that separates a separation target from a liquid, the separation device including: a pump capable of causing a first liquid containing an antibody or an aptamer capable of specifically binding to an antigen of the separation target and a second liquid containing the separation target to pass through a base material made of a porous polyester or a porous polyurethane capable of binding to the antibody or the aptamer; a first valve capable of opening and closing a first flow path through which the first liquid flows; a second valve capable of opening and closing a second flow path through which the second liquid flows; and a controller that controls operation of the pump, opening and closing of the first valve, and opening and closing of the second valve, in which the controller fixes the antibody or the aptamer to a surface of the base material by operating the pump in a state where the first valve is opened and the second valve is closed to cause the first liquid to pass through the base material, and captures the separation target with the antibody or the aptamer by operating, after fixing the antibody or the aptamer to the surface of the base material, the pump in a state where the first valve is closed and the second valve is opened to cause the second liquid to pass through the base material. According to the present invention, a specific cell can be separated from a plurality of types of cells having similar sizes, and similarly, a specific protein can be separated from a plurality of types of proteins having similar sizes.

Hereinafter, embodiments for carrying out the present invention will be described in detail. Note that the present invention is not limited only to the following embodiments, and various modifications can be made within the scope of claims. In addition, the embodiments described in the present specification can be arbitrarily combined with each other to form another embodiment.

Throughout the present specification, expression of a singular should be understood to include also a concept of a plural thereof unless otherwise stated. Thus, an article of a singular (for example, "a", "an", and "the" in English) should be understood to include also a concept of a plural thereof unless otherwise stated. In addition, terms used in the present specification should be understood to be used in a sense commonly used in the art unless otherwise stated. Therefore, unless otherwise defined, all technical and scientific terms used in the present specification have the same meanings as commonly understood by a person skilled in the art to which the present invention belongs. In a case of contradiction, priority is given to the present specification (including definitions).

In addition, in the present specification, a range from "X to Y" includes X and Y, and indicates "X or more and Y or less". Unless otherwise specified, operations and measurements of physical properties and the like are performed at room temperature (20 to 25°C) and at relative humidity of 40 to 60% RH.

In the present specification, the term "(meth)acryl" represents both acryl and methacryl. Therefore, for example, the term "(meth)acrylic acid" encompasses both acrylic acid and methacrylic acid. Similarly, the term "(meth)acryloyl" encompasses both acryloyl and methacryloyl. Therefore, for example, the term "(meth)acryloyl group" encompasses both an acryloyl group and a methacryloyl group.

In the present specification, "A and/or B" means both A and B, or either A or B.

In addition, in the present specification, when a constituent unit is defined to be "derived" from a certain monomer, it means that the constituent unit is a divalent constituent unit generated by cleavage of one bond of a polymerizable unsaturated double bond of the corresponding monomer.

[1 Configuration of filter 10]

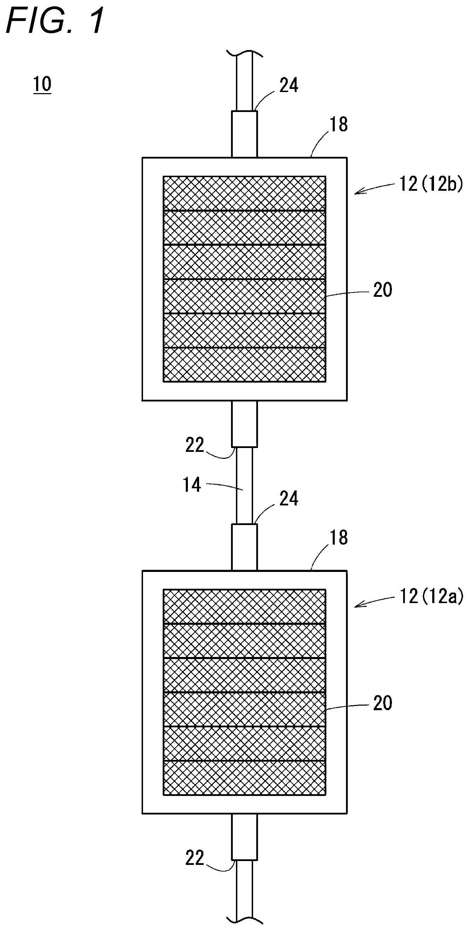

Fig. 1 is a diagram illustrating a configuration of a filter 10. The filter 10 introduces a liquid from an inlet, captures one or more types of separation targets contained in the liquid, and discharges the liquid from an outlet. The liquid is, for example, a cell suspension (cell liquid). The separation target is a cell or a protein. The filter 10 includes one or more filter units 12. The filter 10 illustrated in Fig. 1 includes two filter units 12 (a first filter unit 12a and a second filter unit 12b).

The two filter units 12 are arranged in a line from an upstream side to a downstream side of a flow of a liquid introduced into the filter 10 such that the liquid passes through the filter units 12 in turn. In Fig. 1, the liquid flows from bottom to top. A separation target captured by the first filter unit 12a is different from a separation target captured by the second filter unit 12b. Each of the filter units 12 includes a container 18 and a plurality of base materials 20.

The container 18 accommodates a plurality of the base materials 20. The container 18 has an inlet port 22 and an outlet port 24. The inlet port 22 can introduce the liquid from the outside to the inside of the container 18. The inlet port 22 of the first filter unit 12a corresponds to an inlet of the filter 10. The outlet port 24 can discharge the liquid from the inside to the outside of the container 18. The outlet port 24 of the second filter unit 12b corresponds to an outlet of the filter 10.

The outlet port 24 of the first filter unit 12a and the inlet port 22 of the second filter unit 12b communicate with each other by a connection flow path 14.

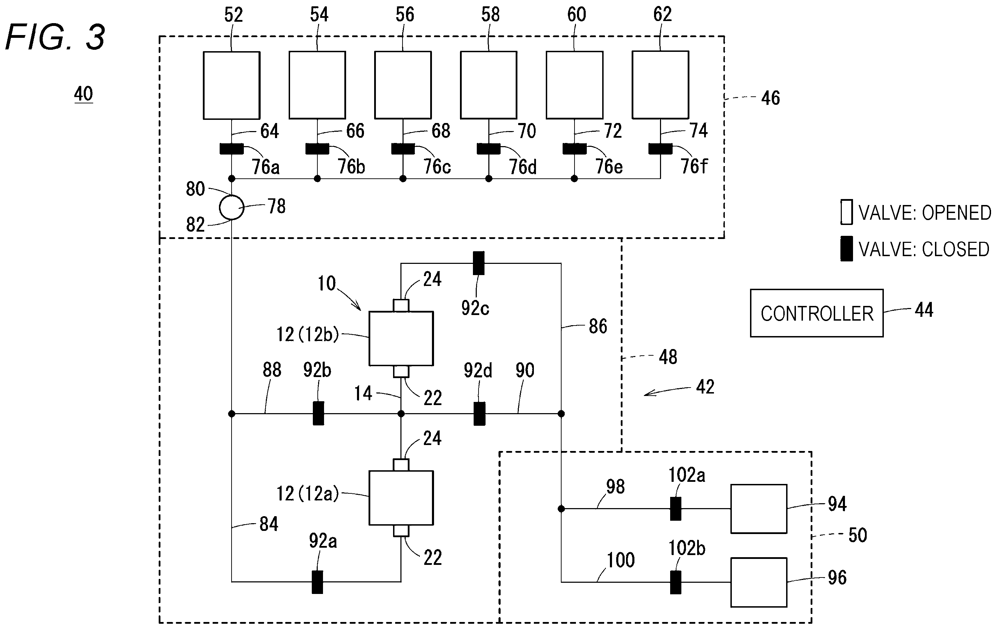

Note that, as illustrated in Fig. 3, by joining of another flow path (first bypass flow path 88) to the connection flow path 14, the inlet port 22 of the second filter unit 12b can be an inlet of the filter 10. As illustrated in Fig. 3, branching of another flow path (second bypass flow path 90) from the connection flow path 14, the outlet port 24 of the first filter unit 12a can be an outlet of the filter 10.

A plurality of the base materials 20 is filled in the container 18. The plurality of base materials 20 is stacked in a thickness direction of the base materials 20. Furthermore, a stacking direction of a plurality of the base materials 20 (thickness direction of the base materials 20) is the same as a direction from the inlet port 22 toward the outlet port 24. That is, a plurality of the base materials 20 is arranged in a line from an upstream side to a downstream side of a flow of the liquid.

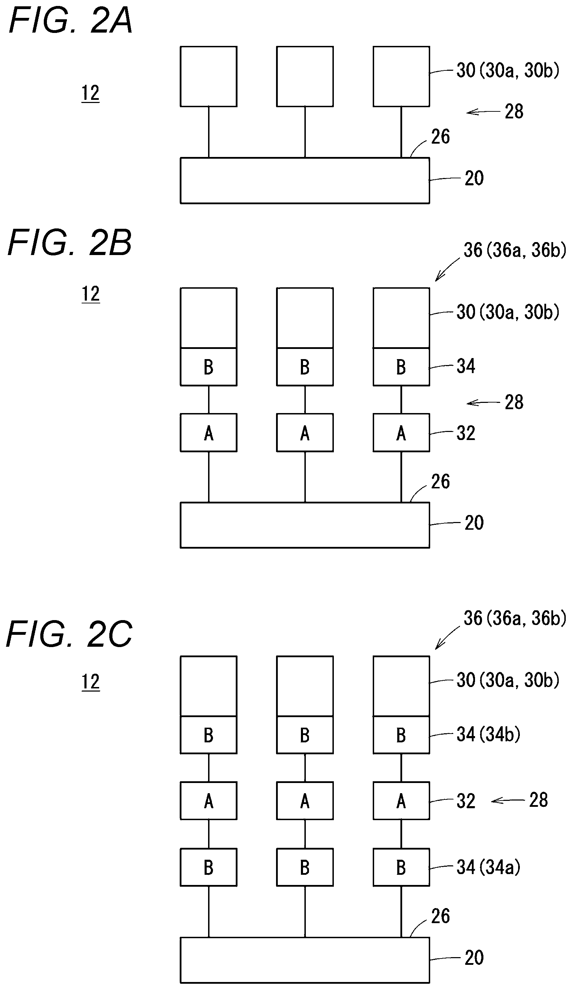

Figs. 2A to 2C are partially enlarged diagrams of the filter unit 12. Each of the drawings schematically illustrates a surface 26 of the base material 20 and an active group 28. The surface 26 of the base material 20 refers to a portion of the base material 20 that can be in contact with a liquid passing through the filter unit 12. The base material 20 is made of a porous polyester or a porous polyurethane having a plurality of micropores. The porous polyester or the porous polyurethane can increase a surface area of the base material 20.

The active group 28 capable of specifically binding to a separation target is fixed to the surface 26 of the base material 20. The active group 28 includes an antibody 30 capable of specifically binding to an antigen included in the separation target. Note that the active group 28 may include an aptamer instead of the antibody 30.

For example, as in the filter unit 12 illustrated in Fig. 2A, the antibody 30 may be directly fixed to the surface 26 of the base material 20. Alternatively, as in the filter unit 12 illustrated in Fig. 2B, a biotinylated antibody 36 in which the antibody 30 and biotin 34 bind to each other and avidin 32 (biotin-binding protein) may be used. For example, the biotinylated antibody 36 may be fixed to the surface 26 of the base material 20 via the avidin 32. Note that another biotin-binding protein such as streptavidin may be used instead of the avidin 32. In the filter unit 12 illustrated in Fig. 2B, the avidin 32 is directly fixed to the surface 26 of the base material 20. Furthermore, the avidin 32 and the biotinylated antibody 36 bind to each other.

As in the filter unit 12 illustrated in Fig. 2C, the biotinylated antibody 36 in which the antibody 30 and a second biotin 34b bind to each other, the avidin 32 (biotin-binding protein), and a first biotin 34a may be used. For example, the biotinylated antibody 36 may be fixed to the surface 26 of the base material 20 via the avidin 32 and the first biotin 34a. In the filter unit 12 illustrated in Fig. 2C, the first biotin 34a is directly fixed to the surface 26 of the base material 20. Furthermore, the first biotin 34a and the avidin 32 bind to each other. In addition, the avidin 32 and the biotinylated antibody 36 bind to each other.

According to the filter units 12 illustrated in Figs. 2A to 2C, when a cell liquid flows from the inlet port 22 toward the outlet port 24 of the filter unit 12, the antibody 30 of the active group 28 binds to an antigen included in a separation target in the cell liquid. The type of the antibody 30 is selected in advance according to the type of the separation target. As a result, while the separation target in the cell liquid is captured by the base material 20, the cell liquid containing substances other than the separation target is discharged from the outlet port 24 to the outside of the filter unit 12.

[2 Method for manufacturing filter unit 12]

As described above, a porous polyurethane is used as the base material 20 of the filter 10. Generally, the porous polyurethane is used as a leukocyte removing filter. Micropores of the porous polyurethane used as the base material 20 are larger than micropores of the leukocyte removing filter. A size of the micropore of the porous polyurethane is adjusted so as to be larger than that of a separation target contained in a cell liquid. The porous polyurethane is cut into a size that can be accommodated in the container 18 to be used as the base material 20. The active group 28 is fixed to the surface 26 of the cut base material 20 by the following manufacturing method.

[2.1 Manufacturing method 1]

The filter unit 12 illustrated in Fig. 2A can be manufactured, for example, as follows. The cut base material 20 and acrylic acid are subjected to a plasma treatment. As a result, an acrylic group is introduced into the surface 26 of the base material 20. Next, a coupling agent (hexamethylenediamine) and the antibody 30 are reacted with the acrylic group on the surface 26 of the base material 20. Alternatively, the cut base material 20 and a compound having an epoxy group (preferably a glycidyl group), such as glycidyl (meth)acrylate, are subjected to a plasma treatment. As a result, an epoxy group (preferably a glycidyl group) is introduced into the surface 26 of the base material 20. Next, a coupling agent (hexamethylenediamine) and the antibody 30 are reacted with the epoxy group (preferably glycidyl group) on the surface 26 of the base material 20. As a result, as illustrated in Fig. 2A, the active group 28 (antibody 30) is fixed to the surface 26 of the base material 20. The base materials 20 to each of which the active group 28 is fixed are stacked and filled in the container 18. Through the above treatment, the filter unit 12 illustrated in Fig. 2A is manufactured.

[2.2 Manufacturing method 2]

The filter unit 12 illustrated in Fig. 2B can be manufactured, for example, as follows. The cut base material 20 and acrylic acid are subjected to a plasma treatment. As a result, an acrylic group is introduced into the surface 26 of the base material 20. Next, a coupling agent (hexamethylenediamine) and the avidin 32 are reacted with the acrylic group on the surface 26 of the base material 20. Alternatively, the cut base material 20 and a compound having an epoxy group (preferably a glycidyl group), such as glycidyl (meth)acrylate, are subjected to a plasma treatment. As a result, an epoxy group (preferably a glycidyl group) is introduced into the surface 26 of the base material 20. Next, a coupling agent (hexamethylenediamine) and the avidin 32 are reacted with the epoxy group (preferably glycidyl group) on the surface 26 of the base material 20. As a result, the avidin 32 is fixed to the surface 26 of the base material 20.

The base materials 20 to each of which the avidin 32 is fixed are stacked and filled in the container 18. Next, an antibody liquid is introduced into the container 18. The antibody liquid includes the biotinylated antibody 36. The avidin 32 fixed to the base material 20 and the biotinylated antibody 36 in the antibody liquid bind to each other. As a result, as illustrated in Fig. 2B, the active group 28 (avidin 32 and biotinylated antibody 36) is fixed to the surface 26 of the base material 20. Through the above treatment, the filter unit 12 illustrated in Fig. 2B is manufactured.

[2.3 Manufacturing method 3]

The filter unit 12 illustrated in Fig. 2B can also be manufactured, for example, as follows. The cut base material 20 is subjected to a plasma treatment. As a result, the surface 26 of the base material 20 is in an active state. Next, the base material 20 is immersed in a solution of a compound having an epoxy group (preferably a glycidyl group), such as glycidyl methacrylate (GMA) or glycidyl acrylate. Alternatively, the base material 20 is brought into contact with a gas of a compound having an epoxy group (preferably a glycidyl group), such as glycidyl methacrylate (GMA) or glycidyl acrylate. As a result, the compound such as GMA binds to the surface 26 of the base material 20. Next, a coupling agent (hexamethylenediamine) and the avidin 32 are reacted with a (meth)acrylic group or the epoxy group on the surface 26 of the base material 20. As a result, the avidin 32 is fixed to the surface 26 of the base material 20. A series of treatments after the avidin 32 is fixed is the same as the series of treatments after the avidin 32 is fixed in the manufacturing method 2.

[2.4 Manufacturing method 4]

The filter unit 12 illustrated in Fig. 2C can be manufactured, for example, as follows. The surface 26 of the cut base material 20 is coated with a polymer having the first biotin 34a binding thereto. As a result, the first biotin 34a is fixed to the surface 26 of the base material 20. The base materials 20 to each of which the first biotin 34a is fixed are stacked and filled in the container 18. Next, an avidin liquid is introduced into the container 18. The avidin liquid contains the avidin 32. The first biotin 34a fixed to the base material 20 and the avidin 32 in the avidin liquid bind to each other. As a result, the avidin 32 is fixed to the surface 26 of the base material 20. A series of treatments after the avidin 32 is fixed is the same as the series of treatments after the avidin 32 is fixed in the manufacturing method 2.

[2.5 Manufacturing method 5]

The filter unit 12 illustrated in Fig. 2C can be also manufactured, for example, as follows. Here, in the filter unit 12 illustrated in Fig. 2C, the active group includes a conjugate in which the antibody or the aptamer binds to second biotin, first biotin fixed to the base material, and a biotin-binding protein capable of binding to each of the first biotin and the second biotin.

Here, the first biotin 34a in Fig. 2C is preferably fixed to the base material via a constituent unit A binding to the base material and derived from a compound A having an epoxy group and an ethylenically unsaturated group, and a constituent unit B derived from a diamine compound B binding to a terminal of the constituent unit A.

That is, in a preferred embodiment of the present invention, a separation device is provided in which biotin is fixed to the base material via a constituent unit A derived from a compound A having an epoxy group and an ethylenically unsaturated group and a constituent unit B derived from a diamine compound B binding to a terminal of the constituent unit A, the constituent unit A binds to the base material, and the constituent unit B binds to the biotin.

The filter unit 12 having such a structure is preferably manufactured by (1) irradiating at least a part of a surface of a base material with plasma (irradiation step), (2) bringing a compound A having an epoxy group and an ethylenically unsaturated group into contact with the base material after the plasma irradiation to bind (fix) the compound A to the base material (compound A fixing step); (3) bringing a diamine compound B into contact with the base material after the compound A is brought into contact with the base material to bind (fix) the diamine compound B to the surface of the base material via a constituent unit A derived from the compound A (diamine compound B fixing step); and (4) bringing biotin (first biotin 34a in Fig. 2C) into contact with the surface of the base material after the diamine compound B is brought into contact with the base material to bind (fix) the biotin to the surface of the base material via the constituent unit A derived from the compound A and the diamine compound B (biotin fixing step).

Therefore, the present invention also provides a method for manufacturing a filter, the method including: (1) irradiating at least a part of a surface of a base material made of a porous polyurethane having a plurality of micropores with plasma, (2) bringing a compound A having an epoxy group and an ethylenically unsaturated group into contact with the base material after the plasma irradiation to bind (fix) the compound A to the base material; (3) bringing a diamine compound B into contact with the base material after the compound A is brought into contact with the base material to bind (fix) the diamine compound B to the surface of the base material via a constituent unit A derived from the compound A; and (4) bringing biotin (first biotin 34a in Fig. 2C) into contact with the surface of the base material after the diamine compound B is brought into contact with the base material to bind (fix) the biotin to the surface of the base material via the constituent unit A derived from the compound A and the diamine compound B.

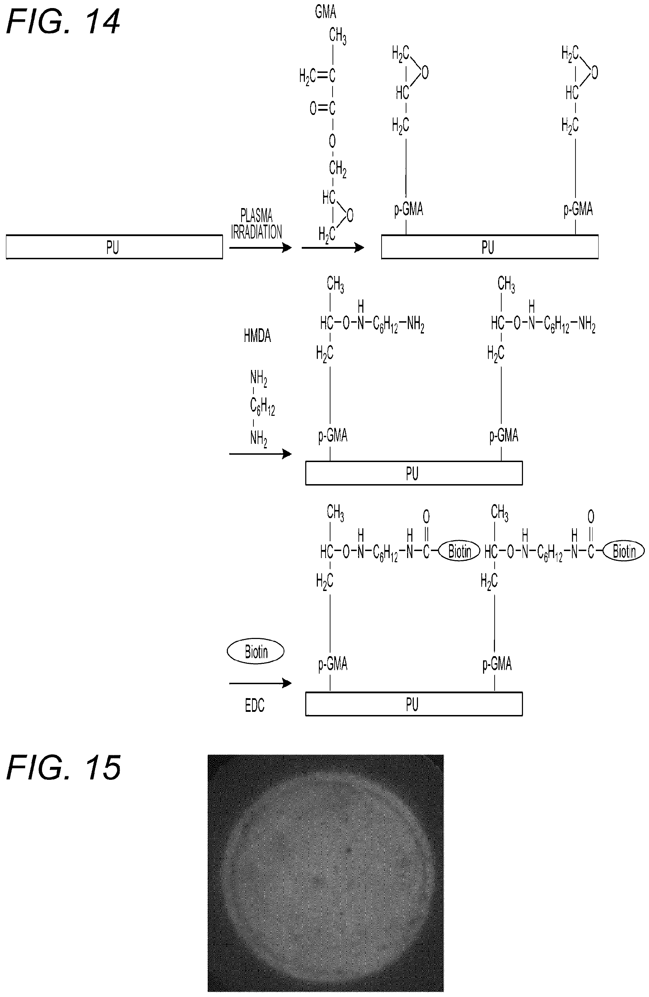

Hereinafter, the above manufacturing method will be described in detail with reference to Fig. 14.

-Irradiation step-

In this step, at least a part of a surface of a base material (base material 20) made of a porous polyurethane having a plurality of micropores is irradiated with plasma. Here, a reason why "at least a part of a surface of a base material is irradiated with plasma" is that it is not always necessary to fix biotin (therefore, an antibody or an aptamer) to all surfaces of the base material (entire surface) in some applications, and it is only required to fix biotin (therefore, an antibody or an aptamer) only to a surface portion (which may be a part or the whole) where it is required to capture a desired cell. Therefore, the plasma irradiation portion preferably includes at least a surface portion where it is required to capture a desired cell, and more preferably substantially includes a surface portion where it is required to capture a desired cell. Note that the phrase "the plasma irradiation portion substantially includes a surface portion where it is required to capture a desired cell" means that the plasma irradiation portion is preferably slightly larger than a surface where it is required to capture the desired cell, and preferably about 0.5 mm or more larger, and more preferably about 10 to 20 mm larger than a margin (outermost circumference) of the surface in order to more reliably capture the desired cell.

The base material 20 is cut into a predetermined size (base material A-1). Here, the base material is made of a porous polyurethane having a plurality of micropores. The size of the base material is not particularly limited, and can be appropriately selected according to an application.

Next, the cut base material (base material A-1) is subjected to a plasma treatment (base material A-2). As a result, a surface of the base material A-1 is in an active state. Therefore, when the surface of the base material A-1 is brought into contact with a compound A having an ethylenically unsaturated group in a next step, the compound A is graft-polymerized with the base material A-1. As a result, the compound A can firmly bind (fixed) to the base material 20. Note that a desired plasma treatment can be performed even on a narrow inner surface having a small diameter, and therefore the plasma irradiation step is preferably performed by an ionization gas plasma treatment.

Before the surface of the base material A-1 is irradiated with plasma, the surface of the base material may be cleaned by an appropriate method. The cleaning method is not particularly limited, and can be performed by cleaning the surface with an appropriate solvent (for example, ultrasonic cleaning, a method for immersing the surface in a cleaning solvent, or a method for pouring a cleaning solvent over the surface).

A pressure condition in the plasma treatment is not particularly limited, and can be either reduced pressure or atmospheric pressure. When the base material is tubular, the entire circumference of the base material (object to be treated) may be uniformly subjected to the plasma treatment without unevenness by irradiating the base material with a plasma gas while rotating a plasma irradiation nozzle around the base material (object to be treated) by one turn.

An ionization gas that can be used for the plasma treatment is one or more gases selected from the group consisting of helium, neon, argon, krypton, air, oxygen, carbon dioxide, carbon monoxide, water vapor, nitrogen, hydrogen, and the like. The plasma treatment is preferably performed in an atmosphere substantially not containing oxygen, and more preferably performed in an atmosphere of at least one inert gas selected from the group consisting of helium, argon, and nitrogen. As a result, the compound A can more efficiently bind (be graft-polymerized) to the base material in a next step. Here, the "atmosphere substantially not containing oxygen" means that the amount of oxygen in the atmosphere is less than 3% by volume, and preferably 0.1% by volume or less. The above atmosphere can be adjusted by introducing a sufficient amount of a predetermined gas into a plasma irradiation device.

Note that plasma treatment conditions (irradiation time, LF output, overcurrent, gas flow rate, gap between electrodes, and plasma irradiation distance) are not particularly limited, and can be appropriately selected according to a binding property (ease of fixation) between the compound A and the base material, the type and area of the base material to be used, and the like.

As an example, irradiation time in the plasma treatment is preferably 5 to 60 seconds, and particularly preferably 10 to 40 seconds. Under such conditions, a sufficient amount of the compound A can be bound (fixed) to the surface of the base material.

As an example, the LF output in the plasma treatment is preferably 100 to 500 W.

As an example, the gas pressure in the plasma treatment is preferably 20 to 60 Pa.

The temperature of the object to be treated (porous polyurethane base material) in the plasma treatment is not particularly limited, and heating or cooling may be performed in addition to room temperature. The plasma treatment is preferably performed at a temperature at which a heating device or a cooling device is unnecessary (for example, 5 to 35°C) from an economical point of view.

A plasma irradiation device (system) that can be used for the plasma treatment is not particularly limited. Examples thereof include a plasma irradiation device (system) including a plasma generation tube that generates plasma by introducing gas molecules and exciting the gas molecules, and an electrode that excites the gas molecules in the plasma generation tube, in which plasma is emitted from one end of the plasma generation tube, but are not limited to such a configuration (system) at all. Examples thereof include devices adopting a high frequency induction method, a capacity-coupled electrode method, a corona discharge electrode-plasma jet method, a parallel plate type, a remote plasma type, an atmospheric pressure plasma type, a low pressure plasma type, and an ICP type high density plasma type. In addition, an ionization gas plasma irradiation device (system), particularly a plasma irradiation device (system) at atmospheric pressure can be used from among those already commercially available. Specifically, a plasma irradiation device manufactured by TRI-STAR TECHNOLOGIES: DURADYNE (product name or trade name), a plasma irradiation device manufactured by DIENER ELECTRONIC: PLASMABEAM, a plasma irradiation device manufactured by DIENER ELECTRONIC: Pico Full PC, or the like can be used, but the plasma irradiation device is not limited thereto at all.

-Compound A fixing step-

In this step, a compound A having an epoxy group and an ethylenically unsaturated group is brought into contact with the base material (base material A-2) that has been irradiated with plasma in the irradiation step. As a result, the compound A is graft-polymerized on the base material in an active state, and the compound A binds (is fixed) to the base material A-2 (base material A-3). Note that, in the present specification, the compound A having an epoxy group and an ethylenically unsaturated group is also simply referred to as "compound A ".

The compound A only needs to have an epoxy group and an ethylenically unsaturated group, and preferably has a glycidyl group and an ethylenically unsaturated group. Examples of such a compound A include a (meth)acrylate having a glycidyl group (epoxy group), such as glycidyl acrylate, glycidyl methacrylate (GMA), 3,4-epoxycyclohexylmethyl acrylate, 3,4-epoxycyclohexylmethyl methacrylate, β-methylglycidyl acrylate, or β-methylglycidyl methacrylate; and a vinyl ether having a glycidyl group (epoxy group), such as allyl glycidyl ether, but are not limited thereto. Among these compounds, the compound A preferably contains at least one selected from the group consisting of glycidyl acrylate, glycidyl methacrylate (GMA), 3,4-epoxycyclohexyl methyl acrylate, 3,4-epoxycyclohexyl methyl methacrylate, β-methyl glycidyl acrylate, and β-methyl glycidyl methacrylate from a viewpoint of, for example, further improving a binding property to the porous polyurethane base material (graft polymerizability with the base material). The compound A is more preferably at least one selected from the group consisting of glycidyl acrylate, glycidyl methacrylate (GMA), 3,4-epoxycyclohexyl methyl acrylate, 3,4-epoxycyclohexyl methyl methacrylate, β-methyl glycidyl acrylate, and β-methyl glycidyl methacrylate.

The compound A still more preferably contains at least one selected from the group consisting of glycidyl acrylate and glycidyl methacrylate. The compound A particularly preferably contains glycidyl acrylate or glycidyl methacrylate, particularly preferably contains glycidyl methacrylate, and is most preferably glycidyl methacrylate. The compound A may be used singly or in combination of two or more types thereof. When two or more types of compounds A are used, constituent units A may be arranged in a block form or randomly.

In this step, as a specific method for bringing the compound A into contact with the base material (base material A-2) after plasma irradiation, either a method for bringing the compound A into contact with the base material A-2 as it is or a method for preparing a compound A solution in which the compound A is dissolved in a solvent and then bringing the compound A solution into contact with the base material A-2 may be used. It is preferable to bring the compound A into contact with the base material A-2 as it is, and it is more preferable to bring the gaseous compound A into contact with the base material A-2 from a viewpoint of, for example, further improving contact efficiency (graft polymerization efficiency) between the base material A-2 and the compound A. The contact between the compound A and the base material A-2 at this time can be performed, for example, by introducing the compound A separately placed under reduced pressure into a plasma irradiation device while the plasma irradiation device is in a reduced pressure state.

In addition, in the method for bringing the compound A solution into contact with the base material A-2, a solvent to be used in dissolving the compound A is not particularly limited, and is appropriately selected according to the type of the compound A. Water (reverse osmosis membrane water (RO water), pure water, deionized water, distilled water, or the like); an alcohol-based solvent such as methanol, ethanol, isopropyl alcohol, or butanol; and an organic solvent such as dichloromethane, chloroform, carbon tetrachloride, tetrahydrofuran (THF), dimethyl sulfoxide, N,N-dimethylformamide (DMF), acetone, dioxane, or benzene are preferably used from a viewpoint of high solubility. These solvents may be used singly or in mixture of two or more types thereof (in a form of a mixed solvent). The concentration of the compound A in the compound A solution is not particularly limited. For example, the concentration of the compound A in the compound A solution is preferably 1 to 10% by mass, and more preferably 2 to 5% by mass. When the concentration of the compound A is within the above range, a sufficient amount of the compound A can be bound (fixed) to the surface of the base material.

Next, the compound A solution prepared as described above is brought into contact with the base material A-2 after plasma irradiation. Here, as a specific method of "contact", a conventionally known method such as a method for applying the compound A solution to the surface of the base material or a method for immersing the base material in the compound A solution can be appropriately adopted.

A contact condition between the compound A or the compound A solution and the base material A-2 may be any condition as long as the compound A can be sufficiently graft-polymerized to the base material A-2. For example, a contact temperature between the compound A or the compound A solution and the base material A-2 is preferably 5 to 50°C, and more preferably 20 to 40°C. In addition, contact time between the compound A or the compound A solution and the base material A-2 is preferably 30 to 90 minutes, more preferably 45 to 70 minutes, and particularly preferably 50 to 60 minutes.

In this step, if necessary, the base material after being brought into contact with the compound A may be cleaned. A cleaning method is not particularly limited, and can be performed by cleaning the base material with an appropriate solvent (for example, water such as ultrafiltered water) (for example, ultrasonic cleaning, a method for immersing the base material in a cleaning solvent, a method for press-cleaning the base material with a roller, or a method for pouring a cleaning solvent over the base material). Cleaning time is not particularly limited, but is preferably 5 to 15 hours. The cleaning treatment may be repeated a plurality of times (for example, 2 to 5 times) as necessary. Note that when the cleaning treatment is repeated a plurality of times, the cleaning treatments may be the same as or different from each other.

-Diamine compound B fixing step-

In this step, a diamine compound B is brought into contact with the base material (base material A-3) having the compound A bound (fixed) to a surface thereof in the compound A fixing step. As a result, the epoxy group (preferably glycidyl group) present in the compound A is ring-opened and reacts with one amino group (-NH$_2$) present in the diamine compound B, and the diamine compound B binds (is fixed) to the base material via the compound A (base material A-4).

The diamine compound B only needs to have two amino groups (-NH$_2$). Examples of such a diamine compound B include ethylenediamine, propanediamine, hexamethylenediamine (HMDA), 1,4-butanediamine, cyclohexanediamine, methylcyclohexanediamine, isophoronediamine, p-phenylenediamine, m-phenylenediamine, p-xylylenediamine, m-xylylenediamine, 4,4'-diaminodiphenylmethane, 4,4'-diaminodiphenyl ether, 1,6-hexanediamine, 1,7-heptanediamine, 1,8-octanediamine, 1,9-nonanediamine, 1,10-decanediamine, 1,11-undecanediamine, 1,12-dodecanediamine, 1,13-tridecanediamine, 1,18-octadecanediamine, 1-butyl-1,2-ethanediamine, 1,1-dimethyl-1,4-butanediamine, 1-ethyl-1,4-butanediamine, 1,2-dimethyl-1,4-butanediamine, 1,3-dimethyl-1,4-butanediamine, 1,4-dimethyl-1,4-butanediamine, 2,3-dimethyl-1,4-butanediamine, 2-methyl-1,5-pentanediamine, 3-methyl-1,5-pentanediamine, 2,5-dimethyl-1,6-hexanediamine, 2,4-dimethyl-1,6-hexanediamine, 3,3-dimethyl-1,6-hexanediamine, 2,2-dimethyl-1,6-hexanediamine, 2,2,4-trimethyl-1,6-hexanediamine, 2,4,4-trimethyl-1,6-hexanediamine, 2,4-diethyl-1,6-hexanediamine, 2,2-dimethyl-1,7-heptanediamine, 2,3-dimethyl-1,7-heptanediamine, 2,4-dimethyl-1,7-heptanediamine, 2,5-dimethyl-1,7-heptanediamine, 2-methyl-1,8-octanediamine, 3-methyl-1,8-octanediamine, 4-methyl-1,8-octanediamine, 1,3-dimethyl-1,8-octanediamine, 1,4-dimethyl-1,8-octanediamine, 2,4-dimethyl-1,8-octanediamine, 3,4-dimethyl-1,8-octanediamine, 4,5-dimethyl-1,8-octanediamine, 2,2-dimethyl-1,8-octanediamine, 3,3-dimethyl-1,8-octanediamine, 4,4-dimethyl-1,8-octanediamine, and 5-methyl-1,9-nonanediamine, but are not limited thereto. Among these compounds, the diamine compound B preferably contains at least one selected from the group consisting of ethylenediamine, trimethylenediamine, 1,2-diaminopropane, tetramethylenediamine, 1,3-diaminobutane, 2,3-diaminobutane, pentamethylenediamine, 2,4-diaminopentane, hexamethylenediamine (HMDA), octamethylenediamine, nonamethylenediamine, decamethylenediamine, undecamethylenediamine, dodecamethylenediamine, tridecamethylenediamine, octadecamethylenediamine, xylylenediamine, and phenylenediamine from a viewpoint of, for example, further improving reactivity with the compound A (therefore, a binding property of the diamine compound B to the base material). The diamine compound B is more preferably at least one selected from the group consisting of ethylenediamine, trimethylenediamine, 1,2-diaminopropane, tetramethylenediamine, 1,3-diaminobutane, 2,3-diaminobutane, pentamethylenediamine, 2,4-diaminopentane, hexamethylenediamine (HMDA), octamethylenediamine, nonamethylenediamine, decamethylenediamine, undecamethylenediamine, dodecamethylenediamine, tridecamethylenediamine, octadecamethylenediamine, xylylenediamine, and phenylenediamine. The diamine compound B more preferably contains at least one selected from the group consisting of hexamethylenediamine (HMDA) and octamethylenediamine. The diamine compound B is most preferably hexamethylenediamine (HMDA). The diamine compound B may be used singly or in combination of two or more types thereof.

In this step, as a specific method for bringing the diamine compound B into contact with the base material (base material A-3) after being brought into contact with the compound A, either a method for bringing the diamine compound B into contact with the base material A-3 as it is or a method for preparing a diamine compound B solution in which the diamine compound B is dissolved in a solvent and then bringing the diamine compound B solution into contact with the base material A-3 may be used. It is preferable to adopt a method for bringing the diamine compound B solution into contact with the base material A-3 from a viewpoint of, for example, further improving contact efficiency between the base material A-3 and the diamine compound B (reactivity between a ring-opened epoxy group and an amino group).

The solvent used for preparing the diamine compound B solution is not particularly limited as long as it can dissolve the diamine compound B, and examples thereof include one or two or more selected from water (reverse osmosis membrane water (RO water), ultrafiltered water, pure water, deionized water, distilled water, or the like); an alcohol such as ethanol, isopropanol, n-propanol, n-butanol, isobutanol, sec-butanol, t-butanol, ethylene glycol, diethylene glycol, propylene glycol, or dipropylene glycol; and an organic solvent such as chloroform, tetrahydrofuran, acetone, dioxane, or benzene, but are not limited thereto. In addition, the concentration of the diamine compound B contained in the diamine compound B solution is not particularly limited, but as an example, the concentration of the diamine compound B in the diamine compound B solution is preferably 0.5 to 5 g/100 mL solvent, more preferably 1 to 3 g/100 mL solvent, and particularly preferably 1 to 2 g/100 mL solvent. At such a concentration, the compound A and the diamine compound B are sufficiently in contact with each other, and therefore a ring-opened epoxy group of the compound A and an amino group of the diamine compound B can be more efficiently reacted.

Next, the diamine compound B solution prepared as described above is brought into contact with the base material A-3. Here, as a specific method of "contact", a conventionally known method such as a method for applying the diamine compound B solution to a surface of the base material or a method for immersing the base material in the diamine compound B solution can be appropriately adopted.

When the contact by the immersion method is performed, the contact may be performed while the diamine compound B solution is stirred. This can further promote the contact between the diamine compound B solution and the base material A-3. The stirring can be performed using a stirring bar or using a known apparatus such as an orbital shaker, a rotary shaker, or a paint shaker. A stirring condition is not particularly limited. A stirring speed is, for example, 200 to 600 rpm, and preferably 300 to 400 rpm from a viewpoint that the diamine compound B solution can be more efficiently brought into contact with the object to be treated. Stirring time is, for example, 0.5 to 24 hours, and preferably 1 to 12 hours from a similar viewpoint. Note that the temperature of the compound B solution at the time of contact by the immersion method may be about room temperature (for example, 20°C or higher and lower than 40°C), but heating (for example, 40 to 60°C) may be performed.

When the diamine compound B is bound to only a part of the base material, the diamine compound B can be bound to a desired surface site of the base material by immersing only the part of the base material in the diamine compound B solution.

When it is difficult to immerse only a part of the base material in the diamine compound B solution, the diamine compound B can be bound to a desired surface site of the base material by previously protecting (covering or the like) a surface portion of the base material to which the diamine compound B does not need to be bound with an appropriate member or material capable of being attached and detached, immersing the base material in the diamine compound B solution, and then detaching the protective member (material). Note that the present invention is not limited to these formation methods, and the diamine compound B can be bound by appropriately using a conventionally known method. For example, when it is difficult to immerse only a part of the base material in the diamine compound B solution, another coating method (for example, a method for applying the diamine compound B solution to a predetermined surface portion of the base material using a spray device or a coating device such as a bar coater, a die coater, a reverse coater, a comma coater, a gravure coater, a spray coater, or a doctor knife) may be applied instead of the immersion method.

A contact condition between the diamine compound B or the diamine compound B solution and the base material A-3 may be any condition as long as the diamine compound B sufficiently reacts with the compound A to bind to the base material A-3. For example, a contact temperature between the diamine compound B or the diamine compound B solution and the base material A-3 is preferably 50 to 90°C, more preferably 60 to 80°C, and particularly preferably 60 to 70°C. In addition, contact time between the diamine compound B or the diamine compound B solution and the base material A-3 is preferably 3 to 25 hours, more preferably 5 to 20 hours, and particularly preferably 8 to 15 hours.

In this step, if necessary, the base material after being brought into contact with the diamine compound B may be cleaned. A cleaning method is not particularly limited, and can be performed by cleaning the base material with an appropriate solvent (for example, water such as ultrafiltered water, or an aqueous hydrochloric acid solution) (for example, ultrasonic cleaning, a method for immersing the base material in a cleaning solvent, a method for press-cleaning the base material with a roller, or a method for pouring a cleaning solvent over the base material). Cleaning time is not particularly limited, but is preferably 5 to 15 hours. The cleaning treatment may be repeated a plurality of times (for example, 2 to 5 times) as necessary. Note that when the cleaning treatment is repeated a plurality of times, the cleaning treatments may be the same as or different from each other.

-Biotin fixing step-

In this step, biotin is brought into contact with a surface of the base material (base material A-4) after being brought into contact with the diamine compound B obtained in the diamine compound B fixing step. As a result, a carboxyl group (-COOH) of the biotin reacts with the other amino group (-NH$_2$) (which has not reacted with the ring-opened epoxy group of the compound A) of the diamine compound B, and the biotin is bound (fixed) to the surface of the base material via the constituent unit A derived from the compound A and the diamine compound B.

In this step, as a specific method for bringing the biotin into contact with the base material (base material A-4) after being brought into contact with the diamine compound B, either a method for bringing the biotin into contact with the base material A-4 as it is or a method for preparing a biotin solution in which the biotin is dissolved in a solvent and then bringing the biotin solution into contact with the base material A-4 may be used. It is preferable to adopt a method for bringing the biotin solution into contact with the base material A-4 from a viewpoint of, for example, further improving contact efficiency between the base material A-4 and the biotin (reactivity between an amino group and a carboxyl group).

The solvent used for preparing the biotin solution is not particularly limited as long as it can dissolve the biotin, and examples thereof include one or two or more selected from water (reverse osmosis membrane water (RO water), ultrafiltered water, pure water, deionized water, distilled water, or the like); an alcohol such as ethanol, isopropanol, n-propanol, n-butanol, isobutanol, sec-butanol, t-butanol, ethylene glycol, diethylene glycol, propylene glycol, or dipropylene glycol; and an organic solvent such as chloroform, tetrahydrofuran, acetone, dioxane, or benzene, but are not limited thereto. In addition, the concentration of the biotin contained in the biotin solution is not particularly limited, but as an example, the concentration of the biotin in the biotin solution is preferably 0.01 to 0.30 g/100 mL solvent, and more preferably 0.05 to 0.15 g/100 mL solvent. At such a concentration, the diamine compound B and the biotin are sufficiently in contact with each other, and therefore an amino group of the diamine compound B and a carboxyl group of the biotin can be more efficiently reacted.

The biotin solution preferably further contains a reaction accelerator for the purpose of improving reaction of the diamine compound B. When the reaction accelerator is used, the reaction accelerator is not particularly limited, and examples thereof include a carbodiimide such as 1-ethyl-(3-dimethylaminopropyl)-carbodiimide•HCl (EDC), dicyclohexylcarbodiimide (DDC), N,N'-diisopropylcarbodiimide (DIC), N,N'-di-tert-butylcarbodiimide, N,N'-dicyclohexylcarbodiimide (DCC), or N-tert-butyl-N-ethylcarbodiimide.

When the reaction accelerator is used, the addition amount of the reaction accelerator may be any amount that can promote reaction between an amino group of the diamine compound B and a carboxyl group of biotin, and can be appropriately selected according to, for example, the type of the diamine compound B or the reaction accelerator. For example, the biotin solution contains the reaction accelerator in a ratio of preferably 100 to 250 parts by mass, more preferably 150 to 200 parts by mass with respect to 100 parts by mass of biotin.

Next, the biotin solution prepared as described above is brought into contact with the base material A-4. Here, as a specific method of "contact", a conventionally known method such as a method for applying the biotin solution to the surface of the base material or a method for immersing the base material in the biotin solution can be appropriately adopted.

When the contact by the immersion method is performed, the contact may be performed while the biotin solution is stirred. This can further promote the contact between the biotin solution and the base material A-4. The stirring can be performed using a stirring bar or using a known apparatus such as an orbital shaker, a rotary shaker, or a paint shaker. A stirring condition is not particularly limited. A stirring speed is, for example, 200 to 600 rpm, and preferably 300 to 400 rpm from a viewpoint that the diamine compound B solution can be more efficiently brought into contact with the object to be treated. Stirring time is, for example, 0.5 to 24 hours, and preferably 1 to 12 hours from a similar viewpoint. Note that the temperature of the biotin solution at the time of contact by the immersion method may be about room temperature (for example, 20°C or higher and lower than 40°C), but heating (for example, 40 to 60°C) may be performed.

When biotin is bound to only a part of the base material, the biotin can be bound to a desired surface site of the base material by immersing only the part of the base material in the biotin solution.

When it is difficult to immerse only a part of the base material in the biotin solution, biotin can be bound to a desired surface site of the base material by previously protecting (covering or the like) a surface portion of the base material to which biotin does not need to be bound with an appropriate member or material capable of being attached and detached, immersing the base material in the biotin solution, and then detaching the protective member (material). Note that the present invention is not limited to these formation methods, and biotin can be bound by appropriately using a conventionally known method. For example, when it is difficult to immerse only a part of the base material in the biotin solution, another coating method (for example, a method for applying the biotin solution to a predetermined surface portion of the base material using a spray device or a coating device such as a bar coater, a die coater, a reverse coater, a comma coater, a gravure coater, a spray coater, or a doctor knife) may be applied instead of the immersion method.

A contact condition between biotin or the biotin solution and the base material A-4 may be any condition as long as biotin sufficiently reacts with the compound A to bind to the base material A-4. For example, a contact temperature between biotin or the biotin solution and the base material A-4 is preferably 5 to 60°C, and more preferably 15 to 35°C. In addition, contact time between biotin or the biotin solution and the base material A-4 is preferably 1 to 30 hours, more preferably 3 to 20 hours, and particularly preferably 5 to 15 hours.

In this step, if necessary, the base material after being brought into contact with biotin may be cleaned. A cleaning method is not particularly limited, and can be performed by cleaning the base material with an appropriate solvent (for example, water such as ultrafiltered water, or an aqueous hydrochloric acid solution) (for example, ultrasonic cleaning, a method for immersing the base material in a cleaning solvent, a method for press-cleaning the base material with a roller, or a method for pouring a cleaning solvent over the base material). Cleaning time is not particularly limited, but is preferably 5 to 15 hours.

The cleaning treatment may be repeated a plurality of times (for example, 2 to 5 times) as necessary. Note that when the cleaning treatment is repeated a plurality of times, the cleaning treatments may be the same as or different from each other.

A filter is obtained as described above. Note that the filter thus obtained can be sterilized by a conventionally known method such as high-pressure steam sterilization, radiation sterilization, ethylene oxide sterilization, or nitrogen dioxide sterilization.

[3 Configuration of separation device 40]

By causing a cell liquid to flow through the filter 10 illustrated in Fig. 1, a desired separation target can be separated from the cell liquid. A device that performs such a treatment is referred to as a separation device 40.

Fig. 3 is a diagram illustrating a configuration of the separation device 40. The separation device 40 can separate a desired separation target from a cell liquid. Furthermore, the separation device 40 can perform some of treatments of the method for manufacturing the filter unit 12 illustrated in Fig. 2C. The separation device 40 includes a separation circuit 42 and a controller 44.

The separation circuit 42 includes a supply unit 46, a separation unit 48, and a discharge unit 50. The supply unit 46 includes a cell liquid container 52, an avidin liquid container 54, a first antibody liquid container 56, a second antibody liquid container 58, a release liquid container 60, a cleaning liquid container 62, a first supply flow path 64 to a sixth supply flow path 74, valves 76a to 76f, and a pump 78. Each of the cell liquid container 52 to the cleaning liquid container 62 is, for example, a medical bag obtained by molding a soft resin material into a bag shape. Each of the cell liquid container 52 to the cleaning liquid container 62 may be a tank or the like made of a hard material. Each of the cell liquid container 52 to the cleaning liquid container 62 is detachable from the separation device 40.

The cell liquid container 52 is filled with a cell liquid. The cell liquid is a solution containing two types of separation targets. In the present embodiment, the two types of separation targets are referred to as a first cell and a second cell. The avidin liquid container 54 is filled with an avidin liquid. The avidin liquid is a solution containing the avidin 32. The first antibody liquid container 56 is filled with a first antibody liquid. The first antibody liquid is a solution containing a first biotinylated antibody 36a (Fig. 2C) in which a first antibody 30a and a second biotin 34b bind to each other. The first antibody 30a can specifically bind to an antigen included in the first cell. The second antibody liquid container 58 is filled with a second antibody liquid. The second antibody liquid is a solution containing a second biotinylated antibody 36b (Fig. 2C) in which a second antibody 30b different from the first antibody 30a and the second biotin 34b bind to each other. The second antibody 30b can specifically bind to an antigen included in the second cell. The release liquid container 60 is filled with a release liquid. The release liquid is, for example, a deoxyribonuclease solution. The cleaning liquid container 62 is filled with a cleaning liquid. The cleaning liquid is, for example, water or phosphate buffered saline (PBS).

The cell liquid container 52 is connected to a suction port 80 of the pump 78 via the first supply flow path 64. The first supply flow path 64 has the valve 76a. The avidin liquid container 54 is connected to the suction port 80 of the pump 78 via the second supply flow path 66. The second supply flow path 66 has the valve 76b. The first antibody liquid container 56 is connected to the suction port 80 of the pump 78 via the third supply flow path 68. The third supply flow path 68 has the valve 76c. The second antibody liquid container 58 is connected to the suction port 80 of the pump 78 via the fourth supply flow path 70. The fourth supply flow path 70 has the valve 76d. The release liquid container 60 is connected to the suction port 80 of the pump 78 via the fifth supply flow path 72. The fifth supply flow path 72 has the valve 76e. The cleaning liquid container 62 is connected to the suction port 80 of the pump 78 via the sixth supply flow path 74. The sixth supply flow path 74 has the valve 76f.

The separation unit 48 includes the filter 10, an upstream flow path 84, a downstream flow path 86, a first bypass flow path 88, a second bypass flow path 90, and valves 92a to 92d. The filter 10 illustrated in Fig. 3 has two filter units 12. Each of the filter units 12 is detachable from the separation device 40. That is, the filter unit 12 can be replaced according to the type of a separation target. The separation unit 48 may be detachable from the separation device 40. Alternatively, the filter 10 may be detachable from the separation device 40.

As described above, the filter 10 includes the first filter unit 12a, the second filter unit 12b, and the connection flow path 14. The inlet port 22 of the first filter unit 12a is connected to a discharge port 82 of the pump 78 via the upstream flow path 84. The upstream flow path 84 has the valve 92a. The upstream flow path 84 located on an upstream side of the valve 92a and the connection flow path 14 communicate with each other by the first bypass flow path 88. The first bypass flow path 88 has the valve 92b. The outlet port 24 of the second filter unit 12b is connected to the downstream flow path 86. The downstream flow path 86 has the valve 92c. The downstream flow path 86 located on a downstream side of the valve 92c and the connection flow path 14 communicate with each other by the second bypass flow path 90. The second bypass flow path 90 has the valve 92d.

The discharge unit 50 includes a collection container 94, a waste liquid container 96, a collection flow path 98, a waste liquid flow path 100, and valves 102a and 102b. Each of the collection flow path 98 and the waste liquid flow path 100 is connected to the downstream flow path 86 of the separation unit 48. The collection flow path 98 has the valve 102a. The waste liquid flow path 100 has the valve 102b.

The collection container 94 is connected to the outlet port 24 of the second filter unit 12b via the collection flow path 98 and the downstream flow path 86. The collection container 94 is connected to the outlet port 24 of the first filter unit 12a via the collection flow path 98, the downstream flow path 86, and the second bypass flow path 90.

The waste liquid container 96 is connected to the outlet port 24 of the second filter unit 12b via the waste liquid flow path 100 and the downstream flow path 86. The waste liquid container 96 is connected to the outlet port 24 of the first filter unit 12a via the waste liquid flow path 100, the downstream flow path 86, and the second bypass flow path 90.

The controller 44 includes a processing circuit and various memories (volatile memory, non-volatile memory, and the like). The processing circuit may be a processor such as a CPU. The processing circuit may be an integrated circuit such as an ASIC or an FPGA. The processor can execute various types of treatment by executing a program stored in the nonvolatile memory. Examples of the volatile memory include a RAM. The volatile memory is used as a working memory of the processor. The volatile memory temporarily stores data and the like necessary for treatment or computation. Examples of the nonvolatile memory include a ROM and a flash memory. The non-volatile memory is used as a storage memory. The nonvolatile memory stores a program, a table, a map, and the like.

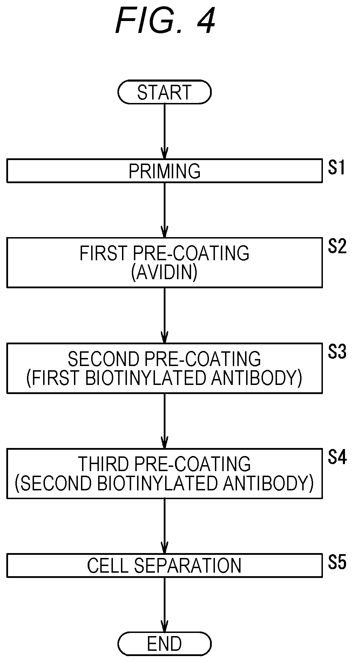

The controller 44 controls opening and closing of each valve of the separation circuit 42. The controller 44 also controls operation of the pump 78. The controller 44 can automatically perform a series of treatments illustrated in Fig. 4 by a program.

[4 Operation of separation device 40]

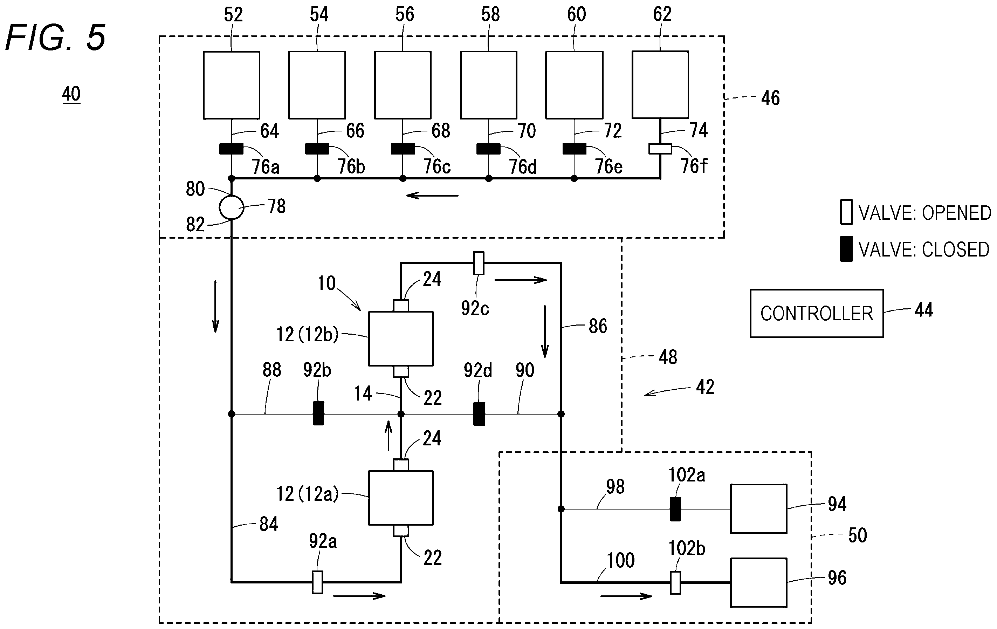

Fig. 4 is a flowchart of a filter manufacturing treatment and a separation treatment performed using the separation device 40. Each of Figs. 5 to 13 is a diagram illustrating opening and closing of a valve of the separation device 40 and a flow of a liquid in the separation device 40 in each step. In Figs. 5 to 13, a black valve means a closed state, and a white valve means an opened state.

Each step illustrated in Fig. 4 is performed by the controller 44 controlling each valve and the pump 78.

A user performs the next preparation work in advance before the series of treatment illustrated in Fig. 4. A user fills each filter unit 12 with the base materials 20 to each of which the first biotin 34a is fixed. The user manufactures the first antibody liquid containing the first biotinylated antibody 36a and fills the first antibody liquid container 56 with the first antibody liquid. The user manufactures the second antibody liquid containing the second biotinylated antibody 36b and fills the second antibody liquid container 58 with the second antibody liquid. In the treatments described with reference to Fig. 4, the first biotinylated antibody 36a and the second biotinylated antibody 36b are substances which can bind to an unnecessary separation target (first cell or second cell) in the cell liquid. The user attaches the cell liquid container 52 to the cleaning liquid container 62 to the separation device 40. When the user operates the controller 44, the series of treatments illustrated in Fig. 4 are started.

In step S1, the controller 44 performs priming. The controller 44 brings the separation circuit 42 into the state illustrated in Fig. 5. That is, the controller 44 opens the valve 76f, the valve 92a, the valve 92c, and the valve 102b and closes the other valves. Furthermore, the controller 44 operates the pump 78. Then, a cleaning liquid in the cleaning liquid container 62 flows through a path indicated by an arrow in Fig. 5 and is collected in the waste liquid container 96.

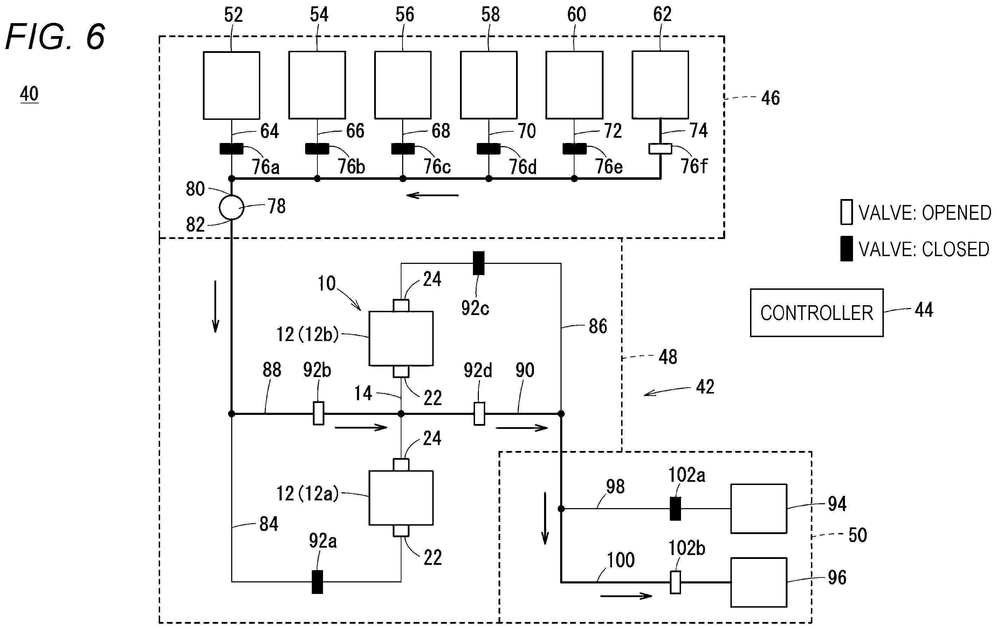

Subsequently, the controller 44 brings the separation circuit 42 into the state illustrated in Fig. 6. That is, the controller 44 opens the valve 76f, the valve 92b, the valve 92d, and the valve 102b and closes the other valves. Furthermore, the controller 44 operates the pump 78. Then, the cleaning liquid in the cleaning liquid container 62 flows through a path indicated by an arrow in Fig. 6 and is collected in the waste liquid container 96. The separation unit 48 is cleaned by the treatment in step S1.

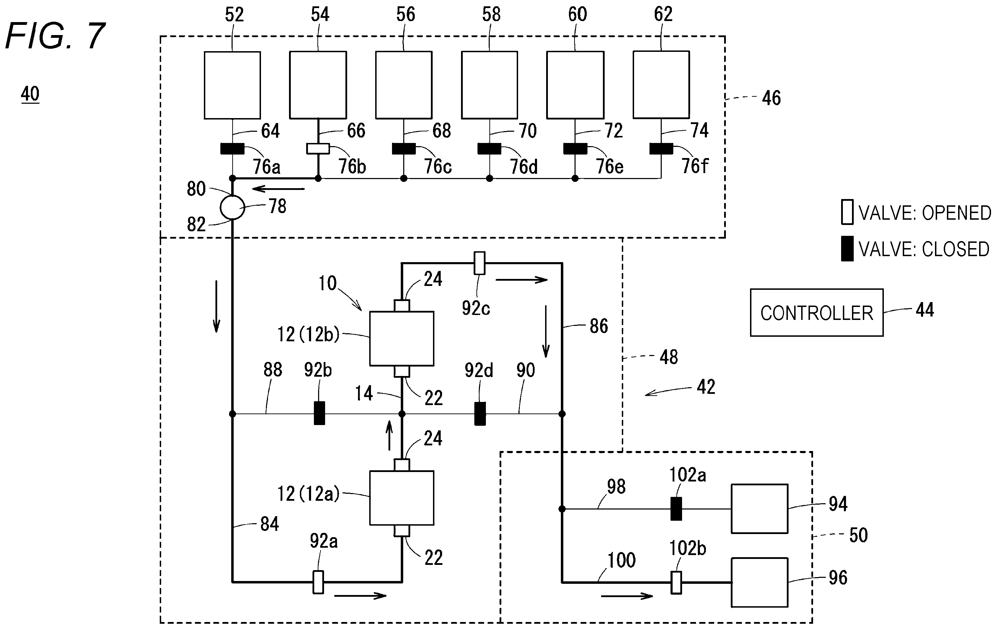

In step S2, the controller 44 performs pre-coating (first pre-coating) of the avidin 32. The controller 44 brings the separation circuit 42 into the state illustrated in Fig. 7. That is, the controller 44 opens the valve 76b, the valve 92a, the valve 92c, and the valve 102b and closes the other valves. Furthermore, the controller 44 operates the pump 78. Then, the avidin liquid in the avidin liquid container 54 flows through a path indicated by an arrow in Fig. 7 and is collected in the waste liquid container 96. The controller 44 operates the pump 78 for a predetermined time or more and then stops the pump 78 for a predetermined time or more. By the treatment in step S2, the avidin liquid is supplied to each filter unit 12, and the base material 20 in the filter unit 12 is immersed in the avidin liquid. The first biotin 34a fixed to the base material 20 binds to the avidin 32 in the avidin liquid. As a result, the avidin 32 is fixed to the base material 20.

Subsequently, the controller 44 brings the separation circuit 42 into the state illustrated in Fig. 5. As a result, a portion through which the avidin liquid flows is cleaned.

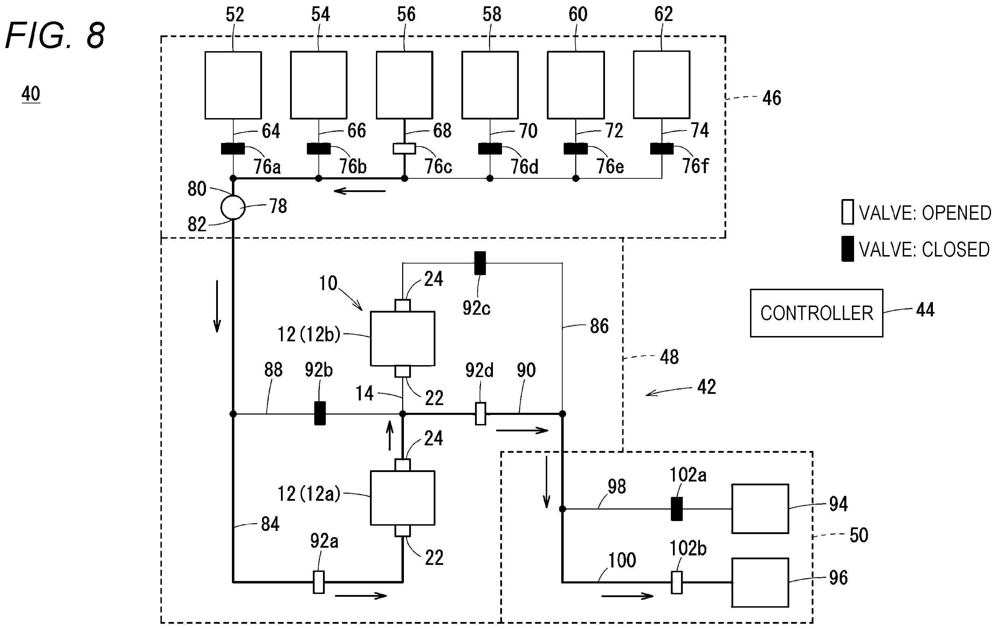

In step S3, the controller 44 performs pre-coating (second pre-coating) of the first biotinylated antibody 36a. Step S3 corresponds to a first step of fixing the first antibody 30a to the base material 20 of the first filter unit 12a. The controller 44 brings the separation circuit 42 into the state illustrated in Fig. 8. That is, the controller 44 opens the valve 76c, the valve 92a, the valve 92d, and the valve 102b and closes the other valves. Furthermore, the controller 44 operates the pump 78. Then, the first antibody liquid in the first antibody liquid container 56 flows through a path indicated by an arrow in Fig. 8 and is collected in the waste liquid container 96. The controller 44 operates the pump 78 for a predetermined time or more and then stops the pump 78 for a predetermined time or more. By the treatment in step S3, the first antibody liquid is supplied to the first filter unit 12a, and the base material 20 in the first filter unit 12a is immersed in the first antibody liquid. The avidin 32 fixed to the base material 20 binds to the first biotinylated antibody 36a in the first antibody liquid. As a result, in the first filter unit 12a, as illustrated in Fig. 2C, the active group 28 (first biotin 34a, avidin 32, and first biotinylated antibody 36a) is fixed to the surface 26 of the base material 20.

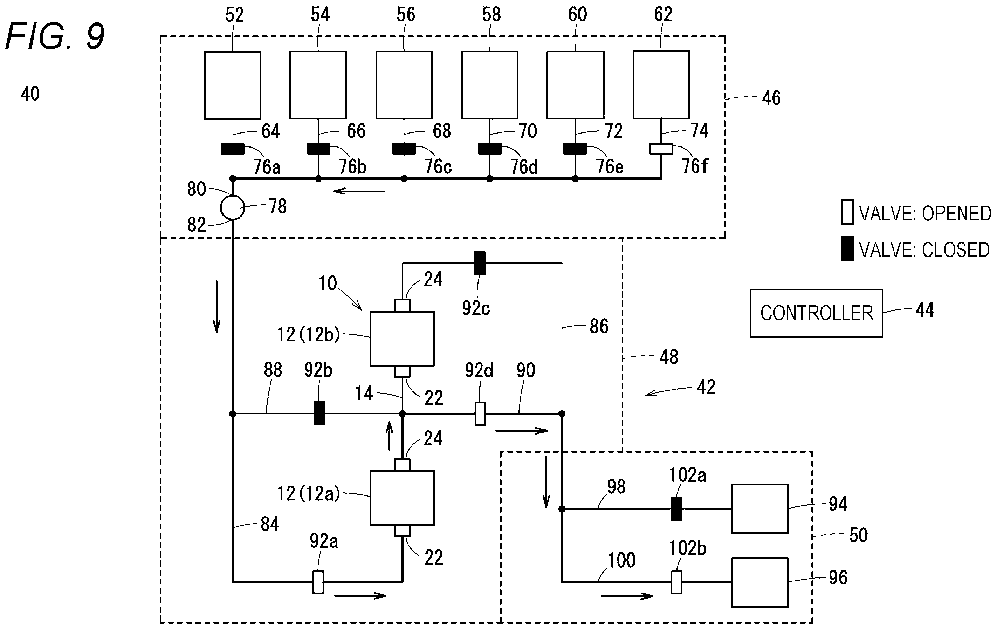

Subsequently, the controller 44 brings the separation circuit 42 into the state illustrated in Fig. 9. That is, the controller 44 opens the valve 76f, the valve 92a, the valve 92d, and the valve 102b and closes the other valves. Furthermore, the controller 44 operates the pump 78. Then, the cleaning liquid in the cleaning liquid container 62 flows through a path indicated by an arrow in Fig. 9 and is collected in the waste liquid container 96. By this treatment, a portion through which the first antibody liquid flows is cleaned.

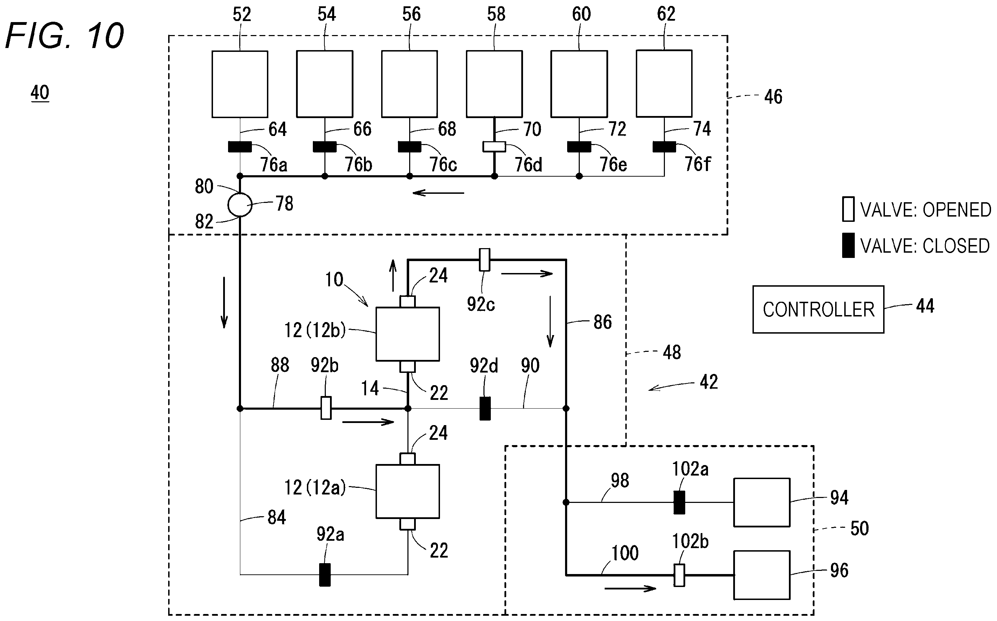

In step S4, the controller 44 performs pre-coating (third pre-coating) of the second biotinylated antibody 36b. Step S4 corresponds to a first step of fixing the second antibody 30b to the base material 20 of the second filter unit 12b. The controller 44 brings the separation circuit 42 into the state illustrated in Fig. 10. That is, the controller 44 opens the valve 76d, the valve 92b, the valve 92c, and the valve 102b and closes the other valves. Furthermore, the controller 44 operates the pump 78. Then, the second antibody liquid in the second antibody liquid container 58 flows through a path indicated by an arrow in Fig. 10 and is collected in the waste liquid container 96. The controller 44 operates the pump 78 for a predetermined time or more and then stops the pump 78 for a predetermined time or more. By the treatment in step S4, the second antibody liquid is supplied to the second filter unit 12b, and the base material 20 in the second filter unit 12b is immersed in the second antibody liquid. The avidin 32 fixed to the base material 20 binds to the second biotinylated antibody 36b in the second antibody liquid. As a result, in the second filter unit 12b, as illustrated in Fig. 2C, the active group 28 (first biotin 34a, avidin 32, and second biotinylated antibody 36b) is fixed to the surface 26 of the base material 20.

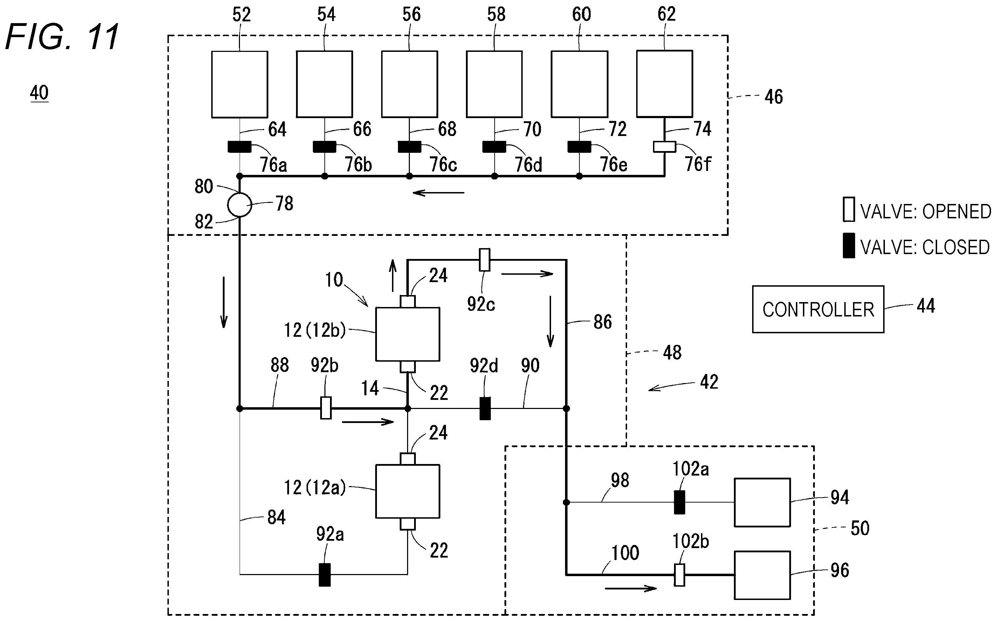

Subsequently, the controller 44 brings the separation circuit 42 into the state illustrated in Fig. 11. That is, the controller 44 opens the valve 76f, the valve 92b, the valve 92c, and the valve 102b and closes the other valves. Furthermore, the controller 44 operates the pump 78. Then, the cleaning liquid in the cleaning liquid container 62 flows through a path indicated by an arrow in Fig. 11 and is collected in the waste liquid container 96. By this treatment, the portion where the second antibody liquid flows is cleaned.

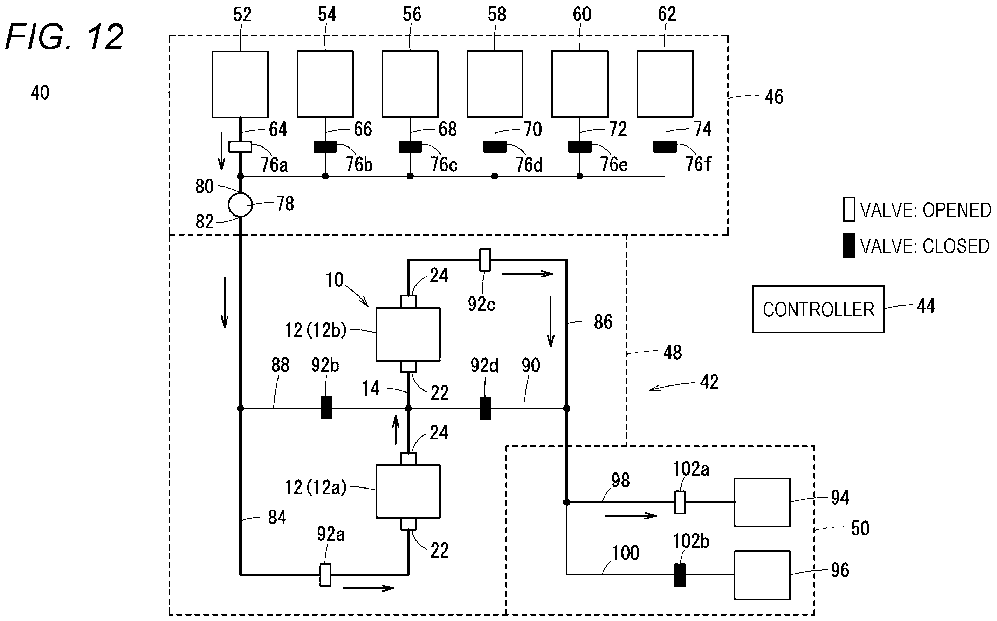

In step S5, the controller 44 separates a desired cell from the cell liquid. Step S5 corresponds to a second step of capturing a separation target. The controller 44 brings the separation circuit 42 into the state illustrated in Fig. 12. That is, the controller 44 opens the valve 76a, the valve 92a, the valve 92c, and the valve 102a and closes the other valves. Furthermore, the controller 44 operates the pump 78. Then, the cell liquid in the cell liquid container 52 flows through a path indicated by the arrow in Fig. 12 and is collected in the collection container 94. The controller 44 operates the pump 78 until the cell liquid in the cell liquid container 52 disappears, and then stops the pump 78. By the treatment in step S5, the cell liquid is supplied to each filter unit 12, and the base material 20 in each filter unit 12 is immersed in the cell liquid. The first biotinylated antibody 36a fixed to the base material 20 of the first filter unit 12a binds to the antigen of the first cell in the cell liquid. The second biotinylated antibody 36b fixed to the base material 20 of the second filter unit 12b binds to the antigen of the second cell in the cell liquid. As a result, in the collection container 94, the cell liquid from which the first cell and the second cell are removed is collected.

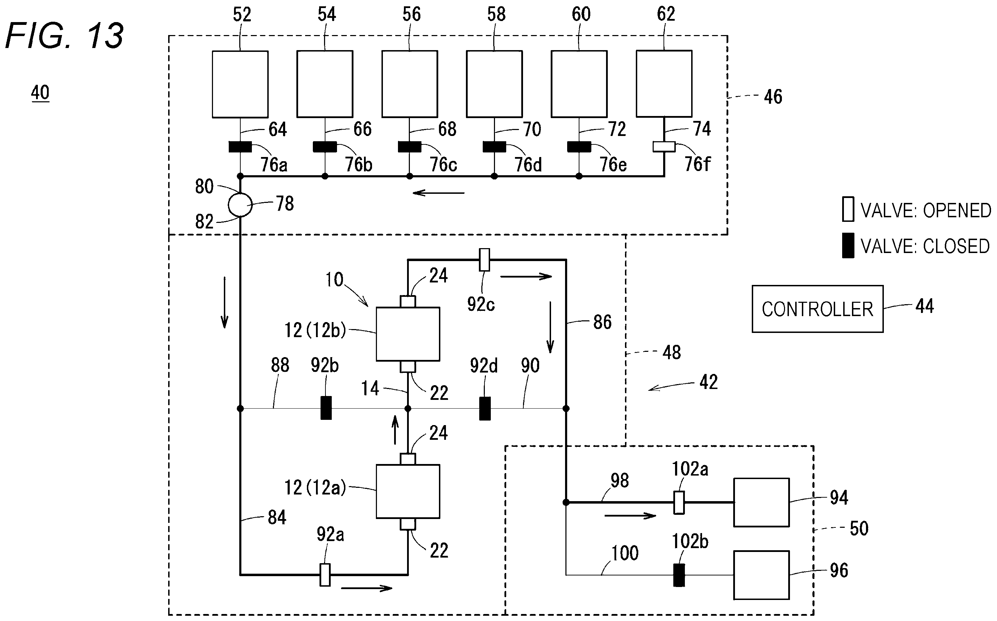

Subsequently, the controller 44 brings the separation circuit 42 into the state illustrated in Fig. 13. As a result, cells remaining in the separation circuit 42 can be collected without waste. This is an end of the series of treatments.

Note that, in the treatments described with reference to Fig. 4, unnecessary separation targets (first cell and second cell) are removed from the cell liquid, and the cell liquid in which desired cells remain is collected. That is, the separation device 40 performs so-called negative selection. Alternatively, it is also possible to extract a necessary separation target from the cell liquid.

That is, the separation device 40 can also perform so-called positive selection. In this case, it is only required to capture a necessary separation target by the filter 10, then to cause the release liquid in the release liquid container 60 to flow to the filter 10, and to collect the release liquid and the separation target in the collection container 94.

[5 Others]

The filter 10 may include another unit that physically separates a separation target. The other unit is made of a porous member and includes another base material different from that of the filter unit 12. A plurality of micropores formed in the other base material is larger than a separation target to be captured by the filter unit 12 and smaller than a separation target to be captured by the other unit.

Examples

Hereinafter, the present invention will be specifically described with reference to Examples, but the present invention is not limited by these Examples. Note that both parts and % in Examples are on a mass basis. Hereinafter, all the conditions for leaving a sample as it is at room temperature without any particular definition are 23°C/55%RH.

Example 1

1. GMA graft polymerization on polyurethane filter medium surface

1) Preparation of base material (base material A-1)
A polyurethane filter medium (manufactured by Terumo Corporation, product name: IMUGARD, thickness: 2 mm) was cut into a size of 15 cm × 5 cm to prepare a PU film.
2) Plasma treatment (base material A-2) and fixation of GMA (base material A-3)

The PU film obtained in the above 1) was allowed to stand in a graft polymerization reaction chamber (plasma irradiation device, Pico Full PC, manufactured by DIENER ELECTRONIC, low pressure type), the chamber was evacuated, and 0.2 torr of Ar gas (100%) was introduced into the chamber. Plasma irradiation (argon ionization gas plasma irradiation) was performed for 40 seconds under conditions of LF output: 400 W and gas pressure: 45 Pa (base material A-2).

Next, a valve between the chamber and a vacuum pump was closed, and a reservoir cock of glycidyl methacrylate (GMA) which had been evacuated in advance was opened to introduce GMA into the chamber.

The chamber was left in this state for one hour, then the pressure in the chamber was returned to normal pressure, and the PU film was taken out from the chamber. As a result, a PU film (GMA-PU film) (base material A-3) having GMA graft-polymerized on a surface thereof was obtained.

2. Fixation of biotin to GMA-PU film (base material A-4)

The GMA-PU film obtained in the above section 1 was impregnated with an HMDA aqueous solution having the following composition, and incubated at 70°C overnight to cause an epoxy group of GMA to react with one amine group of hexamethylenediamine (HMDA) (HMDA-GMA-PU film) (base material A-4).

(Composition of HMDA aqueous solution)

Ultrafiltered water: 186 mL
Ethanol: 14 mL
Hexamethylenediamine (HMDA): 4 g

After completion of the incubation, the HMDA-GMA-PU film was impregnated with a 0.01 N-HCl aqueous solution for eight hours (impregnated HMDA-GMA-PU film).

The impregnated HMDA-GMA-PU film was press-cleaned with a roller while the impregnated HMDA-GMA-PU film was impregnated with 2 L of ultrafiltered water (cleaned HMDA-GMA-PU film).

The cleaned HMDA-GMA-PU film after the press cleaning was impregnated with a biotin aqueous solution having the following composition at room temperature (25°C) overnight to cause the other amine group of hexamethylenediamine (HMDA) to react with a carboxyl group of biotin (biotin-HMDA-GMA-PU film).

(Composition of biotin aqueous solution)

Ultrafiltered water: 200 mL
Biotin: 0.16 g
1-Ethyl-3-(3-dimethylaminopropyl) carbodiimide•HCl(EDC): 0.26 g

After completion of the impregnation, the biotin-HMDA-GMA-PU film was press-cleaned with a roller while the biotin-HMDA-GMA-PU film was impregnated with 2 L of ultrafiltered water (cleaned biotin-HMDA-GMA-PU film).
The cleaned biotin-HMDA-GMA-PU film was left as it was at room temperature (25°C) overnight and was well dried. As a result, a polyurethane filter medium (sample 1) to which biotin bound via GMA and HMDA was obtained.

3. Fluorescence observation

Sample 1 obtained in the above section 2 was punched out to a diameter of 1 mm ($\varphi$1 mm).
Separately, 0.05 (w/v)% of Tween-20 was added to 10 mL of PBS to prepare T-PBS.
Streptavidin-FITC (manufactured by Funakoshi Co., Ltd., trade name: labeled streptavidin (Streptavidin Conjugates), hereinafter "StA-F") was added to 2 mL of PBS at a concentration of 5 $\mu$g/mL to prepare StA-F-PBS.
Sample 1 having a diameter of 1 mm was set in a commercially available filter housing (manufactured by EMD Millipore Corp., trade name: SWINNEX), primed well with T-PBS, and air bubbles in the housing were removed.
2 mL of StA-F-PBS was injected into the housing after priming and incubated at 37°C for 30 minutes.
StA-F not fixed onto sample 1 was cleaned and removed with 30 mL of T-PBS, and then sample 1 was taken out from the housing (measurement sample 1).
Fluorescence observation of measurement sample 1 obtained above was performed under the following conditions using a confocal laser scanning microscope (manufactured by Olympus Corporation, product name: FV3000).

(Observation conditions)

Magnification: 1.25 times
Excitation wavelength: 488 nm
Voltage: 700 V

Results are illustrated in Fig. 15. As illustrated in Fig. 15, sample 1 emitted fluorescence uniformly. From this result, it can be seen that in sample 1, biotin is uniformly fixed onto the polyurethane filter medium.

Example 2

1. Assemble of filter 200

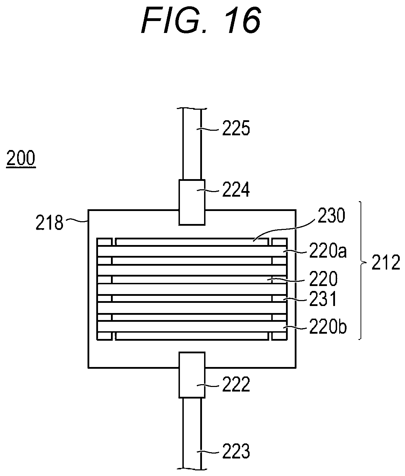

Five biotin-HMDA-GMA-PU films (sample 2) were manufactured in a similar manner to Example 1 except that the polyurethane filter medium (manufactured by Terumo Corporation, product name: IMUGARD, thickness: 2 mm) was cut into a size of 25 mm in diameter ($\Phi$) in Example 1.
For sample 2, cell capture was evaluated according to the following method using a filter 200 illustrated in Fig. 16.
Fig. 16 is a schematic diagram illustrating a cell capture evaluating test device. In Fig. 16, samples 220 as samples 2 were stacked with a silicone spacer 231 (donut shape with inner diameter: 24 mm, outer diameter: 29 mm, and thickness: 1.0 mm) interposed therebetween, and then a polypropylene (PP) mesh (diameter ($\Phi$): 21 mm, thickness: 0.5 mm, mesh size: 1.7 mm $\times$ 1.7 mm)230 was placed at a central portion of each of an uppermost sample 220a as sample 2 and a lowermost sample 220b as sample 2 to prepare a filter unit 212. The filter unit 212 was disposed in a container 218 in which tubes 223 and 225 were connected to a lower part thereof and an upper part thereof via tube connectors 222 and 224, respectively, to assemble the filter 200.

2. Preparation of cell capturing filter

PBS buffer (phosphate buffer, composition: sodium chloride 136.89 mM, potassium chloride 2.68 mM, potassium dihydrogen phosphate 0.147 mM, and disodium hydrogen phosphate (anhydrous) 9.58 mM) was injected from the lower tube 223 of the filter 200 at a flow rate of 1 mL/min using a syringe pump, and the inside of the filter 200 was primed with about 10 mL of PBS buffer.

0.05% (w/v) streptavidin (hereinafter, also referred to as "StA")/PBS solution (StA/PBS solution) was prepared. 5 mL of this StA solution was injected from the lower tube 223 of the filter 200 into the filter 200 at a flow rate of 1 mL/min using a syringe pump. After injection of the StA/PBS solution, the upper tube 225 and the lower tube 223 of the filter 200 were clamped. In this state, incubation was performed at 37°C for 15 minutes.

A porcine CD8 antibody to which biotin bound (manufactured by Thermo Fisher Scientific, product name: Invitrogen, CD8 alpha Monoclonal Antibody (76-2-11), Biotin) was added to PBS so as to have a concentration of 0.005% (w/v) to prepare a porcine CD8 antibody/PBS solution. 5 mL of the porcine CD8 antibody/PBS solution was injected from the lower tube 223 of the filter 200 into the filter 200 at a flow rate of 1 mL/min using a syringe pump. After the injection of the porcine CD8 antibody/PBS solution, the upper tube 225 and the lower tube 223 of the filter 200 were clamped. In this state, incubation was performed at 37°C for 15 minutes.

3. Cell capture evaluation

1000 mL of pig blood was collected into 140 mL of a CPD liquid (composition: sodium citrate hydrate 26.30 g, citric acid hydrate 3.27 g, glucose 23.20 g, sodium dihydrogen phosphate 2.51 g/1,000 mL water for injection) to prepare pig whole blood (CPD blood collection).

Incubation was performed for a predetermined time in the above section 2, and then 5 mL of the whole pig blood (CPD blood collection) was injected from the lower tube 223 of the filter 200 into the filter 200 at a flow rate of 1 mL/min using a syringe pump.

After the injection of the whole pig blood (CPD blood collection), the upper tube 225 and the lower tube 223 of the filter 200 were clamped. In this state, incubation was performed at 37°C for 15 minutes.

After the incubation, PBS buffer was injected from the lower tube 223 of the filter 200 into the filter 200 at a flow rate of 1 mL/min using a syringe pump, and 5 mL of the pig whole blood in the filter 200 was collected.

Blood count measurement and flow site measurement were performed on the pig whole blood before the filter injection and the pig whole blood collected above, and a CD8 positive ratio in the pig whole blood total white blood cells before and after the filter injection was calculated. Based on these values, a CD8 positive cell capture ratio by the filter was calculated according to the following formula.

[Mathematical Formula 1]

$$\text{CD8 positive cell capture ratio} = \left( 1 - \frac{\text{Number of CD8 positive cells in blood after collection}}{\text{Number of CD8 positive cells in blood after injection}} \right) \times 100 \; (\%)$$

Comparative Example 1

A polyurethane filter medium (manufactured by Terumo Corporation, product name: IMUGARD, thickness: 2 mm) was cut into a size of 25 mm in diameter (Φ), and five comparative PU films (Comparative Example 1) were prepared.

A CD8 positive cell capture ratio was calculated in a similar manner to Example 2 except that the comparative PU film was used instead of sample 2 in Example 2.

As a result, sample 2 (Example 2) captured significantly more cells than the comparative PU film (Comparative Example 1).

Note that the present invention is not limited to the above-described disclosure, and can adopt various configurations without departing from the gist of the present invention.

The present application is based on Japanese Application No. 2022-123245 filed on August 2, 2022, the entire content of which is incorporated herein by reference.

Reference Signs List

18    Container (third container)
20    Base material
26    Surface
30    Antibody

| 40 | Separation device |
|----|----|
| 44 | Controller |
| 52 | Cell liquid container (second container) |
| 56 | First antibody liquid container (first container) |
| 58 | Second antibody liquid container (first container) |
| 64 | First supply flow path (second flow path) |
| 68 | Third supply flow path (first flow path) |
| 70 | Fourth supply flow path (first flow path) |
| 76a | Valve (second valve) |
| 76c | Valve (first valve) |
| 76d | Valve (first valve) |
| 78 | Pump |

**Claims**

1. A separation device that separates a separation target from a liquid, the separation device comprising:

    a pump capable of causing a first liquid containing an antibody or an aptamer capable of specifically binding to an antigen of the separation target and a second liquid containing the separation target to pass through a base material made of a porous polyester or a porous polyurethane capable of binding to the antibody or the aptamer;
    a first valve capable of opening and closing a first flow path through which the first liquid flows;
    a second valve capable of opening and closing a second flow path through which the second liquid flows; and
    a controller that controls operation of the pump, opening and closing of the first valve, and opening and closing of the second valve, wherein
    the controller
    fixes the antibody or the aptamer to a surface of the base material by operating the pump in a state where the first valve is opened and the second valve is closed to cause the first liquid to pass through the base material, and
    captures the separation target with the antibody or the aptamer by operating, after fixing the antibody or the aptamer to the surface of the base material, the pump in a state where the first valve is closed and the second valve is opened to cause the second liquid to pass through the base material.

2. The separation device according to claim 1, comprising:

    a first container that stores the first liquid;
    a second container that stores the second liquid; and
    a third container that accommodates the base material, wherein
    the controller
    causes the first container and the third container to communicate with each other by opening the first valve and closing the second valve, and
    causes the second container and the third container to communicate with each other by closing the first valve and opening the second valve after fixing the antibody or the aptamer to the surface of the base material.

3. The separation device according to claim 2, wherein

    the third container accommodates a plurality of the base materials, and
    a plurality of the base materials is stacked from an upstream side to a downstream side of a flow of the first liquid and the second liquid.

4. The separation device according to claim 3, comprising a plurality of the third containers arranged in a line from the upstream side to the downstream side of the flow of the first liquid and the second liquid, wherein
a type of the antibody or the aptamer fixed to the base material is different among the third containers.

5. The separation device according to claim 1, wherein

    biotin is fixed to a surface of the base material via a constituent unit A derived from a compound A having an epoxy group and an ethylenically unsaturated group and a constituent unit B derived from a diamine compound B binding to a terminal of the constituent unit A, and
    the constituent unit A binds to the base material, and the constituent unit B binds to the biotin.

6.  The separation device according to claim 5, wherein
the compound A is at least one selected from the group consisting of glycidyl acrylate, glycidyl methacrylate (GMA), 3,4-epoxycyclohexyl methyl acrylate, 3,4-epoxycyclohexyl methyl methacrylate, β-methyl glycidyl acrylate, and β-methyl glycidyl methacrylate.

7.  The separation device according to claim 5, wherein
the compound B is at least one selected from the group consisting of ethylenediamine, trimethylenediamine, 1,2-diaminopropane, tetramethylenediamine, 1,3-diaminobutane, 2,3-diaminobutane, pentamethylenediamine, 2,4-diaminopentane, hexamethylenediamine (HMDA), octamethylenediamine, nonamethylenediamine, decamethylenediamine, undecamethylenediamine, dodecamethylenediamine, tridecamethylenediamine, octadecamethylenediamine, xylylenediamine, and phenylenediamine.

8.  A separation method for separating a separation target from a liquid, the separation method comprising:

a first step of fixing an antibody or an aptamer capable of specifically binding to an antigen of the separation target to a surface of a base material made of a porous polyester or a porous polyurethane capable of binding to the antibody or the aptamer by causing a first liquid containing the antibody or the aptamer to pass through the base material, and
a second step of capturing the separation target with the antibody or the aptamer by causing a second liquid containing the separation target to pass through the base material after the first step.

9.  The separation method according to claim 8, wherein
a separation device including:

a first container that stores the first liquid;
a second container that stores the second liquid;
a third container that accommodates the base material;
a pump capable of causing the first liquid and the second liquid to flow to the base material;
a first valve capable of opening and closing a first flow path through which the first liquid flows;
a second valve capable of opening and closing a second flow path through which the second liquid flows; and
a controller that controls operation of the pump, opening and closing of the first valve, and opening and closing of the second valve
is used, and
the controller
causes the first container and the third container to communicate with each other by opening the first valve and closing the second valve in the first step, and
causes the second container and the third container to communicate with each other by closing the first valve and opening the second valve in the second step.
FIG. 3

44 CONTROLLER
VALVE: OPENED
VALVE: CLOSED
FIG. 4
S1 PRIMING
S2 FIRST PRE-COATING (AVIDIN)
S3 SECOND PRE-COATING (FIRST BIOTINYLATED ANTIBODY)
S4 THIRD PRE-COATING (SECOND BIOTINYLATED ANTIBODY)
S5 CELL SEPARATION

FIG. 5

44 CONTROLLER
VALVE: OPENED
VALVE: CLOSED

FIG. 6

44 CONTROLLER
VALVE: OPENED
VALVE: CLOSED

FIG. 7

44 CONTROLLER
VALVE: OPENED
VALVE: CLOSED

FIG. 8

44 CONTROLLER
VALVE: OPENED
VALVE: CLOSED

FIG. 9

44 CONTROLLER
VALVE: OPENED
VALVE: CLOSED

FIG. 10

44 CONTROLLER
VALVE: OPENED
VALVE: CLOSED

FIG. 11

44 CONTROLLER
VALVE: OPENED
VALVE: CLOSED

FIG. 12

44 CONTROLLER
VALVE: OPENED
VALVE: CLOSED

FIG. 13

44 CONTROLLER
VALVE: OPENED
VALVE: CLOSED

FIG. 14
PLASMA IRRADIATION

# FIG. 1

## FIG. 2A

## FIG. 2B

## FIG. 2C

FIG. 3

VALVE: OPENED
VALVE: CLOSED

CONTROLLER — 44

# FIG. 4

```
        ( START )
            │
            ▼
┌───────────────────────────────┐  S1
│          PRIMING              │
└───────────────────────────────┘
            │
            ▼
┌───────────────────────────────┐  S2
│      FIRST PRE-COATING        │
│          (AVIDIN)             │
└───────────────────────────────┘
            │
            ▼
┌───────────────────────────────┐  S3
│      SECOND PRE-COATING       │
│  (FIRST BIOTINYLATED ANTIBODY)│
└───────────────────────────────┘
            │
            ▼
┌───────────────────────────────┐  S4
│       THIRD PRE-COATING       │
│ (SECOND BIOTINYLATED ANTIBODY)│
└───────────────────────────────┘
            │
            ▼
┌───────────────────────────────┐  S5
│       CELL SEPARATION         │
└───────────────────────────────┘
            │
            ▼
         ( END )
```

FIG. 5

VALVE: OPENED
VALVE: CLOSED

CONTROLLER — 44

EP 4 563 687 A1

FIG. 6

FIG. 7

40

VALVE: OPENED
VALVE: CLOSED

CONTROLLER 44

FIG. 8

40

VALVE: OPENED
VALVE: CLOSED

CONTROLLER  44

FIG. 9

VALVE: OPENED
VALVE: CLOSED

CONTROLLER — 44

EP 4 563 687 A1

FIG. 10

40

52  54  56  58  60  62

46

64  66  68  70  72  74

76a  76b  76c  76d  76e  76f

80  78

82

VALVE: OPENED

VALVE: CLOSED

CONTROLLER  44

48

42

10

12 (12b)  24  92c

86

88  92b  14  92d  90

22

24

12 (12a)

84  92a

22

98  102a

94

50

100  102b

96

EP 4 563 687 A1

FIG. 11

40

VALVE: OPENED
VALVE: CLOSED

CONTROLLER — 44

EP 4 563 687 A1

FIG. 12

40

VALVE: OPENED

VALVE: CLOSED

CONTROLLER — 44

EP 4 563 687 A1

FIG. 13

VALVE: OPENED
VALVE: CLOSED

CONTROLLER — 44

EP 4 563 687 A1

# FIG. 14

# FIG. 15

# FIG. 16

## INTERNATIONAL SEARCH REPORT

| International application No. |
|---|
| **PCT/JP2023/028254** |

**A. CLASSIFICATION OF SUBJECT MATTER**

***C12M 1/00***(2006.01)i; ***C12M 3/00***(2006.01)i
FI: C12M1/00 Z; C12M3/00 Z

According to International Patent Classification (IPC) or to both national classification and IPC

**B. FIELDS SEARCHED**

Minimum documentation searched (classification system followed by classification symbols)

C12M1/00; C12M3/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 9-49830 A (TERUMO CORP) 18 February 1997 (1997-02-18) | 8 |
|  | claims, examples |  |
| Y | claims, examples | 1-9 |
| X | JP 2020-525795 A (TECHNISCHE UNIVERSITAT DARMSTADT) 27 August 2020 (2020-08-27) | 8 |
|  | claims, paragraphs [0006], [0042]-[0046] |  |
| Y | claims, paragraphs [0006], [0042]-[0046] | 1-9 |
| Y | JP 2009-525728 A (MICROCHIP BIOTECHNOLOGIES, INC.) 16 July 2009 (2009-07-16) | 1-9 |
|  | fig. 3, etc. |  |
| Y | JP 2019-512711 A (HELIXBIND, INC.) 16 May 2019 (2019-05-16) | 1-9 |
|  | claims, etc. |  |
| Y | JP 2022-512328 A (ELEMENT BIOSCIENCES, INC.) 03 February 2022 (2022-02-03) | 1-9 |
|  | claims, etc. |  |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance |  |  |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means |  |  |
| "P" | document published prior to the international filing date but later than the priority date claimed | "&" | document member of the same patent family |

| Date of the actual completion of the international search | Date of mailing of the international search report |
|---|---|
| **03 October 2023** | **10 October 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** |  |
|  | Telephone No. |

Form PCT/ISA/210 (second sheet) (January 2015)

## INTERNATIONAL SEARCH REPORT
### Information on patent family members

International application No.

**PCT/JP2023/028254**

| Patent document<br>cited in search report | Publication date<br>(day/month/year) | Patent family member(s) | Publication date<br>(day/month/year) |
|---|---|---|---|
| JP 9-49830 A | 18 February 1997 | (Family: none) | |
| JP 2020-525795 A | 27 August 2020 | WO 2019/001952 A1<br>claims, pp. 2, 12-13 | |
| JP 2009-525728 A | 16 July 2009 | WO 2008/030631 A2<br>fig. 3 | |
| JP 2019-512711 A | 16 May 2019 | WO 2017/160820 A1<br>claims | |
| JP 2022-512328 A | 03 February 2022 | WO 2020/118255 A1<br>claims | |

Form PCT/ISA/210 (patent family annex) (January 2015)

**REFERENCES CITED IN THE DESCRIPTION**

**Patent documents cited in the description**

- JP 2011024575 A

- JP 2022123245 A